# EUROPEAN PATENT APPLICATION

(11) **EP 4 621 407 A1**
(43) Date of publication of application: **24.09.2025**
(21) Application number: 23903095.0
(22) Date of filing: 16.10.2023
(51) Int. Cl.: G01N 33/48, G01N 1/28, G01N 27/62

(54) **METHOD FOR PREPARING PEPTIDE-CONTAINING SAMPLE**

(30) Priority: 13.12.2022 JP 2022199022; 10.08.2023 JP 2023131671
(71) Applicant: Kazusa DNA Research Institute, Kisarazu-shi, Chiba 292-0818 (JP)
(72) Inventor: KAWASHIMA, Yusuke, Kisarazu-shi, Chiba 292-0818 (JP); OHARA, Osamu, Kisarazu-shi, Chiba 292-0818 (JP); KONNO, Ryo, Kisarazu-shi, Chiba 292-0818 (JP); ISHIKAWA, Masaki, Kisarazu-shi, Chiba 292-0818 (JP); NAKAJIMA, Daisuke, Kisarazu-shi, Chiba 292-0818 (JP)
(74) Representative: J A Kemp LLP
(86) International application number: PCT/JP2023/037460
(87) International publication number: WO 2024/127796

(57) **Abstract**

By adding 2,2-didecylpropane-1,3-bis-β-D-maltopyranoside (LMNG) to an aqueous peptide solution, adsorption of the peptide to a vessel or the like is suppressed. LMNG can be easily removed from the peptide by a reversed-phase columns. In addition, at least one sugar-based nonionic surfactant is added to an aqueous solvent for dissolving dried peptides. The addition of the sugar-based nonionic surfactant significantly accelerates the dissolution of peptides, particularly long peptides. The combination of two sugar-based nonionic surfactants further enhances the acceleration effect on the dissolution of dry peptides. For example, n-dodecyl-β-D-maltoside (DDM) may be combined with α-D-glucopyranosyl-α-D-glucopyranoside monododecanoate (trehalose C12).

## Description

### TECHNICAL FIELD

The present invention widely relates to a technology for minimizing peptide loss in the preparation of a trace peptide-containing sample for use in liquid chromatography-mass spectrometry (LC-MS) analysis and the like. Specifically, the present invention relates to a method for preparing a peptide-containing sample, a method for dissolving dried peptides generated in the pretreatment process of a sample for proteome analysis, and use of a surfactant for promoting the dissolution of the dried peptides. The present invention also relates to a stable aqueous peptide solution.

### BACKGROUND ART

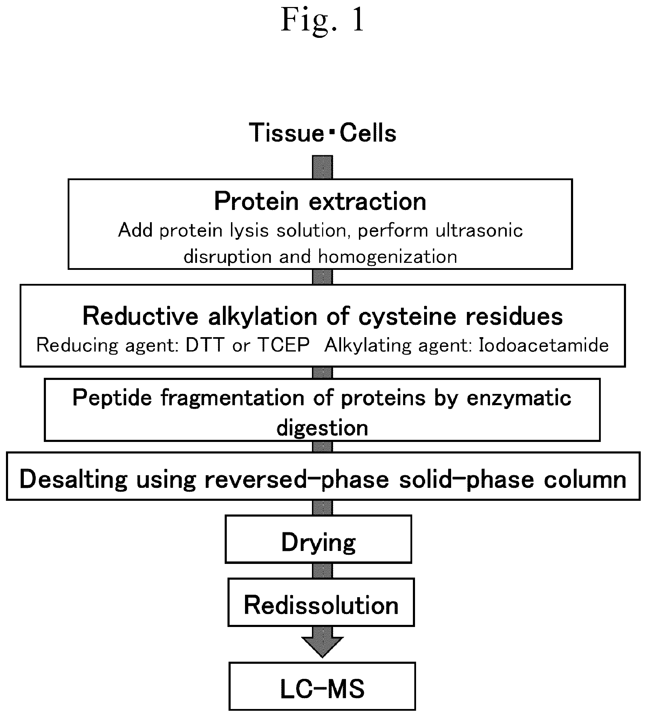

Proteome analysis has entered an era in which 10,000 or more proteins can be observed in a single measurement, and further contributions to the fields of biology and medicine are expected. One way for further improving proteome analysis technology is to reduce peptide loss during sample preparation. The major pretreatment steps for proteome analysis include (1) protein extraction, (2) reductive alkylation, (3) enzyme digestion, (4) desalting by reversed-phase spin column (mainly STAGE-Tip), (5) drying, and (6) redissolution (Figure 1). Efforts have been made to improve recovery rates and throughput in various processes. In recent years, with the arrival of methods such as SP3 and S-Trap, it has become common practice to dissolve proteins from cells or tissues using a powerful surfactant, perform reductive alkylation, remove the surfactant and salts, and replace the solvent with one suitable for digestive enzymes and then digest the proteins. However, in the case of trace samples, peptides may be lost due to non-specific adsorption to tubes or tips during long-term incubation, such as protein digestion, or when transferring digested peptides to another tube.

The use of surfactants is desirable to minimize this loss; however, one problem is that surfactants are trapped in the reversed-phase solid-phase column and are eluted together with the peptides. In the case of trace samples, it is common to dry the extract from the reversed-phase solid-phase column, redissolve the peptide with a trace volume of solvent, and then analyze the concentrated peptides by LC-MS. In doing so, concentrated surfactants are also naturally analyzed at the same time, increasing the risk of sensitivity reduction due to contamination in MS. While there are methods for removing surfactants after digestion by precipitating or decomposing them, performing such treatment increases the number of process steps, which in turn leads to various disadvantages such as peptide loss, reduced reproducibility, and increased labor. Therefore, it is ideal to be able to remove surfactants in a standard process without performing any special operations.

Additionally, drying of peptides promotes non-specific adsorption to tubes or the like, leading to the risk of peptide loss. When sample quantities are small, the risk of peptide loss is particularly high. Considering LC-MS analysis, the solvent used to dissolve dried peptide fragments is required to not interfere with peptide analysis by LC-MS and to cause less contamination to the mass spectrometer. Therefore, a solution of about 2 to 5% (v/v) acetonitrile (ACN) with about 0.1% (v/v) trifluoroacetic acid (TFA) or formic acid is commonly used to dissolve dried peptide fragments, as it is volatile and does not affect the background of MS measurements. However, it is difficult to recover peptide fragments that have been dried and adsorbed nonspecifically to the container with a high yield.

One method for reducing the effect of non-specific adsorption of peptides is to add a carrier protein such as albumin to the sample. However, this adds complexity to the sample preparation, affects the background of MS measurements, and raises concerns about contamination of the mass spectrometer.

One method known to reduce the effect of non-specific adsorption of peptides is to add high concentration of NaCl to the solvent for dissolving the peptides. However, while high concentration of NaCl can suppress adsorption due to ion interactions to a certain extent, their suppressive effect on adsorption due to hydrophobic interactions is limited.

Such peptide loss may be reduced by dissolving the dried peptides in a solvent containing a surfactant; however, surfactants may interfere with peptide analysis by LC-MS due to decreased LC separation efficiency or suppressed ionization. Surfactants suitable for LC-MS include for example, degradable surfactants such as RapiGest SF (trademark) (Non-Patent Document 1) or MaSDeS (Non-Patent Document 2); removable surfactants such as sodium deoxycholate (Non-Patent Documents 3, 4) or sodium dodecanoate (Non-Patent Document 5); and surfactants such as Invitrosol (trademark) (Non-Patent Document 6) whose retention time in LC does not overlap with that of peptides. However, degrading or removing surfactants is a time-consuming treatment, and there is a risk that peptides will be non-specifically adsorbed onto tubes and vials since surfactants no longer remain in the solution after such treatment. When surfactants with a different LC retention time from peptides are used, direct measurement by LC-MS can be performed without any hassle and peptides after dissolution are considered to be more stable; however, Invitrosol is an expensive reagent.

On the other hand, n-dodecyl-β-D-maltoside (DDM) is a nonionic surfactant with a sugar group in its hydrophilic portion, and is mainly used to solubilize membrane proteins. DDM is inexpensive and has been used for pretreatment in single-cell proteome analysis, and has been shown to be effective in preventing the loss of proteins and peptides (Non-Patent Documents 7 and 8). However, its effects on the dissolution of dried peptides have not yet been investigated.

Lauryl maltose neopentyl glycol (LMNG) (2,2-didecylpropane-1,3-bis-β-D- maltopyranoside) is a nonionic surfactant with two linked hydrophobic chains with equal length and two hydrophilic maltoside groups that has been shown to solubilize and stabilize membrane proteins better than DDM.

### PRIOR ART DOCUMENTS

### NON-PATENT DOCUMENTS

Non-Patent Document 1: Mosen, P. R. et al, Proteomics 2021, 21(20), e2100129
Non-Patent Document 2: Chang, Y. H. et al, J Proteome Res 2015, 14(3), 1587-1599
Non-Patent Document 3: Masuda, T. et al, J Proteome Res 2008, 7(2), 731-740
Non-Patent Document 4: Sialana, F. J. et al, J Proteome Res 2022, 21(8), 1842-1856
Non-Patent Document 5: Lin, Y. et al., PLoS One 2013, 8 (3), e59779
Non-Patent Document 6: Kawashima, Y. et al., Proteomics 2013, 13(5), 751-755
Non-Patent Document 7: Liu, J. et al, Anal Chem 2015, 87 (4), 2054-2057
Non-Patent Document 8: Tsai, C. F. et al. Commun Biol 2021, 4 (1), 265

### SUMMARY OF THE INVENTION

### PROBLEMS TO BE SOLVED BY THE INVENTION

The present invention aims to provide a technology for reducing peptide loss while avoiding contamination of LC-MS in the preparation of trace peptide-containing samples for use in proteome analysis and the like. In addition, the present invention also aims to provide a technology for improving the dissolution of dried peptides adsorbed on the wall surface of a tube in the pretreatment process of proteome analysis, while preventing the dissolved peptides from being re-adsorbed non-specifically to the tube or chip.

### MEANS TO SOLVE THE PROBLEMS

The present inventors conducted intensive studies to solve the above problems and found that when LMNG was added to a protein-containing sample during digestion with a protease, the loss of peptides from the sample was suppressed and the number of peptides and proteins identified with LC-MS increased. When LMNG was added during affinity purification of peptides, peptide loss from the sample was also suppressed, and the number of peptides and proteins identified by LC-MS increased. LMNG has a strong affinity for the reversed-phase column, and was retained in the column even under elution conditions in which most peptides could be eluted from the reversed-phase column. As a result, LMNG was successfully removed from the peptides during the desalting process using a reversed-phase column, thereby minimizing the possibility of contamination of the LC-MS instrument.

In addition, by adding a sugar-based nonionic surfactant such as DDM to the solution for recovering dried peptides, the solubility of the dried peptides was improved, and recovery of peptides was successfully improved. In particular, the recovery of long peptide fragments consisting of 11 or more amino acids was greatly improved. Even at DDM concentrations below CMC, an improvement in the recovery of peptide fragments was observed, and this effect was achieved even at concentrations as low as 0.005% (w/w). Surprisingly, by adding a combination of two or more sugar-based nonionic surfactants, an improvement in the solubility of dried peptides exceeding the additive effect was observed. Based on these findings, the present inventors conducted further studies and completed the present invention.

Namely, the present invention relates to the following.
[1] A method for preparing a peptide-containing sample comprising the following steps:
   1) providing an aqueous peptide solution containing a peptide and 2,2-dodecylpropane-1,3-bis-β-D-maltopyranoside (hereinafter referred to as "LMNG");
   2) applying the obtained aqueous peptide solution to a reversed-phase column to allow the peptide and LMNG to be adsorbed onto the reversed-phase column; and
   3) eluting the adsorbed peptide from the reversed-phase column while retaining LMNG adsorbed onto the reversed-phase column to remove LMNG from the peptide.
[2] The preparation method of [1], wherein the reversed-phase column is a C18 column or a styrene-divinylbenzene copolymer column.
[3] The preparation method of [1] or [2], wherein the peptide is eluted from the reversed-phase column with an eluent having a polarity equivalent to or greater than a mixed solution of acetonitrile / 0.1% (v/v) trifluoroacetic acid aqueous solution = 50/50 (v/v).
[4] The preparation method of any of [1] to [3], wherein the polarity of the eluent is equivalent to or less than a mixed solution of acetonitrile / 0.1% (v/v) trifluoroacetic acid aqueous solution = 30/70 (v/v).
[5] The preparation method of any of [1] to [4], wherein the LMNG concentration in the aqueous peptide solution is lower than the critical micelle concentration.
[6] The preparation method of any of [1] to [5], wherein the aqueous peptide solution is provided as contained in a vessel having a plastic surface.
[7] The preparation method of any of [1] to [6], further comprising subjecting a protein dissolved in an aqueous solvent containing LMNG to limited digestion with a protease to obtain an aqueous peptide solution containing a peptide and LMNG.
[8] The preparation method of any of [1] to [7], further comprising applying the aqueous peptide solution provided in step 1) to an affinity chromatography to obtain an aqueous peptide solution containing an affinity-enriched peptide and LMNG.
[9] The preparation method of [8], wherein the affinity chromatography is immobilized metal affinity chromatography or avidin or streptavidin affinity chromatography.
[10] The preparation method of any of [1] to [9], wherein the peptide-containing sample is for LC-MS analysis.
[11] A method for producing an aqueous peptide solution, comprising dissolving a dried peptide in an aqueous solvent containing at least one sugar-based nonionic surfactant.
[12] The production method of [11], wherein the sugar-based nonionic surfactant is a compound represented by formula (I);
   [Chemistry 1]

   G₁ - L₁ - R₁ (I)

   (wherein G₁ is a monosaccharide or disaccharide residue, L₁ is O, S, -O-(CH₂)ₙ-O-, or -O-CO-, R₁ is a linear alkyl group having 6 to 12 carbon atoms, and n is an integer of 1 to 3), or by formula (II); 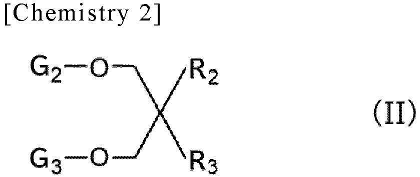 (wherein G₂ and G₃ are the same or different and represent a monosaccharide or disaccharide residue, and R₂ and R₃ are the same or different and represent a linear alkyl group having 6 to 12 carbon atoms).
[13] The production method of [12], wherein the sugar-based nonionic surfactant is a compound represented by formula (I), and G₁ is glucose residue, mannose residue, maltose residue or trehalose residue.
[14] The production method of [13], wherein the sugar-based nonionic surfactant is n-dodecyl-β-D-maltoside, n-undecyl-β-D-maltoside, n-decyl-β-D-maltoside, n-nonyl-β-D-thiomaltoside, n-octyl-β-D-thioglucoside, 3-oxatridecyl-α-D-mannoside, or α-D-glucopyranosyl-α-D-glucopyranoside monododecanoate.
[15] The production method of [12], wherein the sugar-based nonionic surfactant is a compound represented by formula (II), and G₂ and G₃ are the same and represent glucose residue or maltose residue.
[16] The production method of [15], wherein the sugar-based nonionic surfactant is 2,2-dioctylpropane-1,3-bis-β-D-maltopyranoside, 2,2-didecylpropane-1,3-bis-β-D-maltopyranoside, or 2,2-dihexylpropane-1,3-bis-β-D-glucopyranoside.
[17] The production method of any of [11] to [16], wherein the aqueous solvent contains a combination of at least two sugar-based nonionic surfactants.
[18] The production method of [17], wherein the combination of sugar-based nonionic surfactants is selected from the group consisting of:
   n-dodecyl-β-D-maltoside and α-D-glucopyranosyl-α-D-glucopyranoside monododecanoate;
   2,2-dihexylpropane-1,3-bis-β-D-glucopyranoside and α-D-glucopyranosyl-α-D-glucopyranoside monododecanoate;
   2,2-dioctylpropane-1,3-bis-β-D-maltopyranoside and 2,2-dihexylpropane-1,3-bis-β-D-glucopyranoside; and
   3-oxatridecyl-α-D-mannoside and 2,2-dioctylpropane-1,3-bis-β-D-maltopyranoside.
[19] The production method of any of [11] to [18], wherein the dried peptide is contained in a vessel having a plastic surface.
[20] An agent for accelerating the dissolution of a dried peptide, comprising at least one sugar-based nonionic surfactant.
[21] The agent for accelerating the dissolution of a dried peptide of [20], comprising a combination of at least two sugar-based nonionic surfactants.
[22] The agent for accelerating the dissolution of a dried peptide of [21], wherein the combination of sugar-based nonionic surfactants is selected from the following group:
   n-dodecyl-β-D-maltoside and α-D-glucopyranosyl-α-D-glucopyranoside monodododecanoate;
   2,2-dihexylpropane-1,3-bis-β-D-glucopyranoside and α-D-glucopyranosyl-α-D-glucopyranoside monodododecanoate;
   2,2-dioctylpropane-1,3-bis-β-D-maltopyranoside and 2,2-dihexylpropane-1,3-bis-β-D-glucopyranoside; and
   3-oxatridecyl-α-D-mannoside and 2,2-dioctylpropane-1,3-bis-β-D-maltopyranoside.
[23] An aqueous peptide solution, comprising a peptide and a combination of at least two sugar-based nonionic surfactants.
[24] The aqueous peptide solution of [23], wherein the combination of sugar-based nonionic surfactants is any one selected from the following group:
   n-dodecyl-β-D-maltoside and α-D-glucopyranosyl-α-D-glucopyranoside monodododecanoate;
   2,2-dihexylpropane-1,3-bis-β-D-glucopyranoside and α-D-glucopyranosyl-α-D-glucopyranoside monodododecanoate;
   2,2-dioctylpropane-1,3-bis-β-D-maltopyranoside and 2,2-dihexylpropane-1,3-bis-β-D-glucopyranoside; and
   3-oxatridecyl-α-D-mannoside and 2,2-dioctylpropane-1,3-bis-β-D-maltopyranoside.
[25] The preparation method of any of [1] to [10], further comprising the following steps:
   4) drying the eluted peptide to obtain a dried peptide, and
   5) dissolving the obtained dried peptide in an aqueous solvent containing at least one sugar-based nonionic surfactant to obtain an aqueous peptide solution.
[26] The preparation method of [25], wherein the sugar-based nonionic surfactant is a compound represented by formula (I);

   [Chemistry 3]

   G₁ - L₁ - R₁ (I)

   (wherein G₁ is a monosaccharide or disaccharide residue, L₁ is O, S, -O-(CH₂)ₙ-O-, or -O-CO-, R₁ is a linear alkyl group having 6 to 12 carbon atoms, and n is an integer of 1 to 3), or by formula (II); 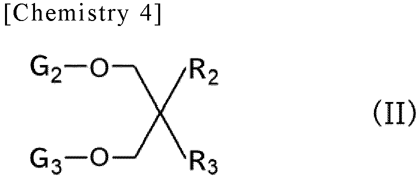 (wherein G₂ and G₃ are the same or different and represent a monosaccharide or disaccharide residue, and R₂ and R₃ are the same or different and represent a linear alkyl group having 6 to 12 carbon atoms).
[27] The preparation method of [26], wherein the sugar-based nonionic surfactant is a compound represented by formula (I) and G₁ is glucose residue, mannose residue, maltose residue or trehalose residue.
[28] The preparation method of [27], wherein the sugar-based nonionic surfactant is n-dodecyl-β-D-maltoside, n-undecyl-β-D-maltoside, n-decyl-β-D-maltoside, n-nonyl-β-D-thiomaltoside, n-octyl-β-D-thioglucoside, 3-oxatridecyl-α-D-mannoside, or α-D-glucopyranosyl-α-D-glucopyranoside monododecanoate.
[29] The preparation method of [26], wherein the sugar-based nonionic surfactant is a compound represented by formula (II), and G₂ and G₃ are the same and are glucose residue or maltose residue.
[30] The preparation method of [29], wherein the sugar-based nonionic surfactant is 2,2-dioctylpropane-1,3-bis-β-D-maltopyranoside, 2,2-didecylpropane-1,3-bis-β-D-maltopyranoside or 2,2-dihexylpropane -1,3-bis-β-D-glucopyranoside.
[31] The preparation method of any of [25] to [30], wherein the aqueous solvent comprises a combination of at least two sugar-based nonionic surfactants.
[32] The preparation method of [31], wherein the combination of sugar-based nonionic surfactants is any one selected from the following group:
   n-dodecyl-β-D-maltoside and α-D-glucopyranosyl-α-D-glucopyranoside monodododecanoate;
   2,2-dihexylpropane-1,3-bis-β-D-glucopyranoside and α-D-glucopyranosyl-α-D-glucopyranoside monodododecanoate;
   2,2-dioctylpropane-1,3-bis-β-D-maltopyranoside and 2,2-dihexylpropane-1,3-bis-β-D-glucopyranoside; and
   3-oxatridecyl-α-D-mannoside and 2,2-dioctylpropane-1,3-bis-β-D-maltopyranoside.
[33] The preparation method of any of [25] to [32], wherein the dried peptide is contained in a vessel having a plastic surface.

### EFFECTS OF THE INVENTION

According to the present invention, it is possible to minimize peptide loss while avoiding contamination of LC-MS in the preparation of a trace peptide-containing sample for use in proteome analysis or the like. By treating a sample for LC-MS analysis using the method of the present invention, the loss of peptides from trace samples is suppressed, the number of peptides and proteins identified by mass analysis is increased, and the reproducibility of analysis is improved. In addition, according to the present invention, the recovery of peptide fragments adsorbed onto the tube wall by drying during the pretreatment process for proteome analysis is improved. In particular, since the recovery of long peptides with a length of 11 amino acids or more is greatly improved, it is possible to prepare a sample for mass spectrometry that is rich in long peptides, thereby increasing the number of peptides and proteins identified by mass spectrometry and contributing to improved accuracy of proteome analysis. Since the effect of improved recovery of peptide fragments can be observed at a very low concentration of sugar-based nonionic surfactants, contamination of the mass spectrometer can also be minimized. Furthermore, according to the present invention, a stable aqueous peptide solution in which adsorption of peptides to the vessel is suppressed.

### BRIEF DESCRIPTION OF THE DRAWINGS

[Figure 1] Figure 1 shows a workflow of sample pretreatment in proteome analysis.
[Figure 2A] Figures 2A to 2D show the evaluation of recovery rates of dried tryptic peptides by DDA-LC-MS/MS. Figure 2A shows TIC chromatograms of dried tryptic peptides from K562 cells, which were dissolved in 2% ACN-0.1% TFA or 0.005-0.04% DDM. Dried peptides (500 ng) in a normal polypropylene (PP) tube were dissolved in 25 µL of each solvent, transferred to a PP LC vial, and 2.5 µL thereof was injected into LC-MS/MS.
[Figure 2B] Figure 2B shows the number of protein groups and peptides identified by DDA-LC-MS/MS.
[Figure 2C] Figure 2C shows the distribution of retention times for the identified peptides.
[Figure 2D] Figure 2D shows the distribution of amino acid lengths of the identified peptides.
[Figure 3A] Figures 3A-D show the evaluation of the recovery of dried peptides in different vials and dissolution solutions by DIA-LC-MS/MS. Figure 3A shows the number of peptides identified from dried peptides dissolved in 2% ACN-0.1% TFA or 0.02% DDM in regular PP or hydrophilic vials. 500 ng of dried peptides in each vial were dissolved in 25 µL of each solvent and 2.5 µL thereof was injected into LC-MS/MS.
[Figure 3B] Figure 3B shows a comparison of total peptide intensities at each amino acid length.
[Figure 3C] Figure 3C shows a Pearson correlation coefficient heatmap with hierarchical clustering of DIA protein intensity.
[Figure 3D] Figure 3D shows the principal component analysis of DIA protein intensity.
[Figure 4A] Figures 4A-D shows the effect of DDM on peptide recovery in IP-MS.

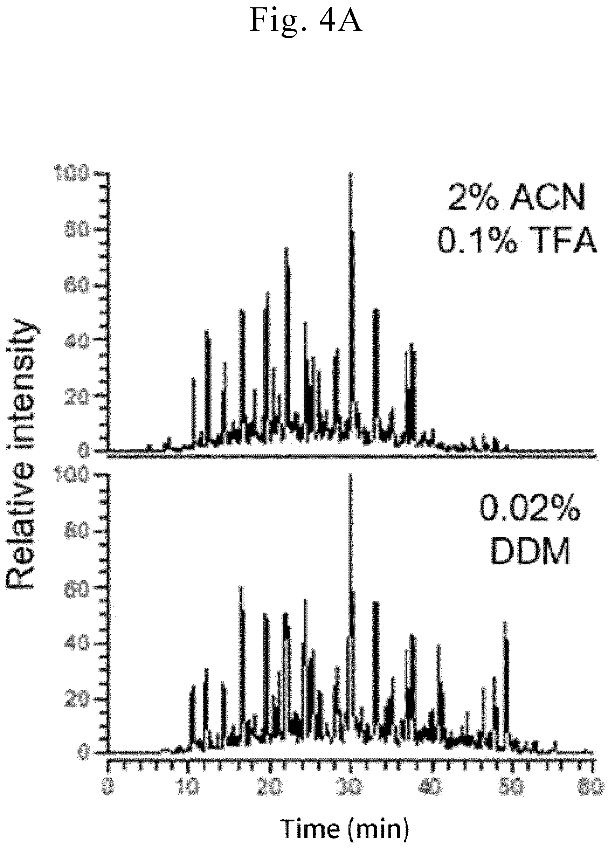

Figure 4A shows TIC chromatograms of dried tryptic peptides in IP of RELA dissolved in 2% ACN-0.1% TFA or 0.02% DDM.

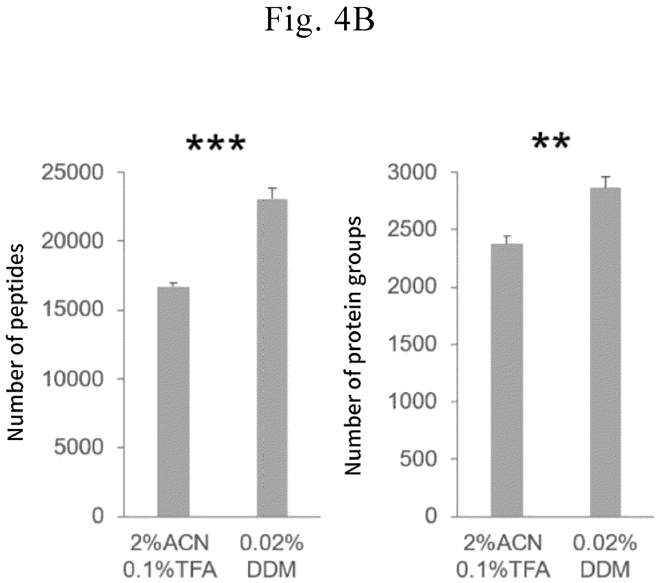

[Figure 4B] Figure 4B shows the number of protein groups and peptides identified by DIA-LC-MS/MS.

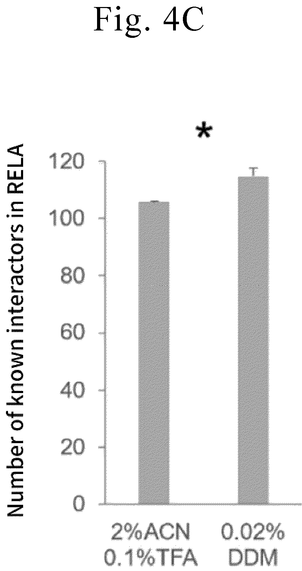

[Figure 4C] Figure 4C shows the number of known RELA interactors in the identified proteins. The known RELA interactors were extracted from IntAct (https://www.ebi.ac.uk/intact/home).

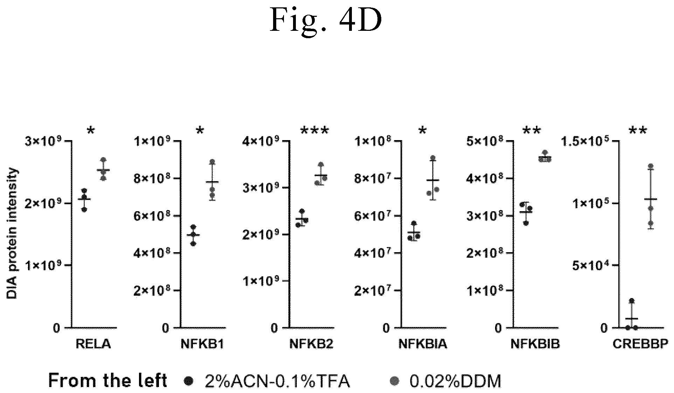

[Figure 4D] Figure 4D shows the DIA protein intensities of RELA and major RELA interactors in the identified proteins. The DIA protein intensitie of 0 indicates a missing value. * p< 0.05; ** p< 0.01; *** p< 0.005.

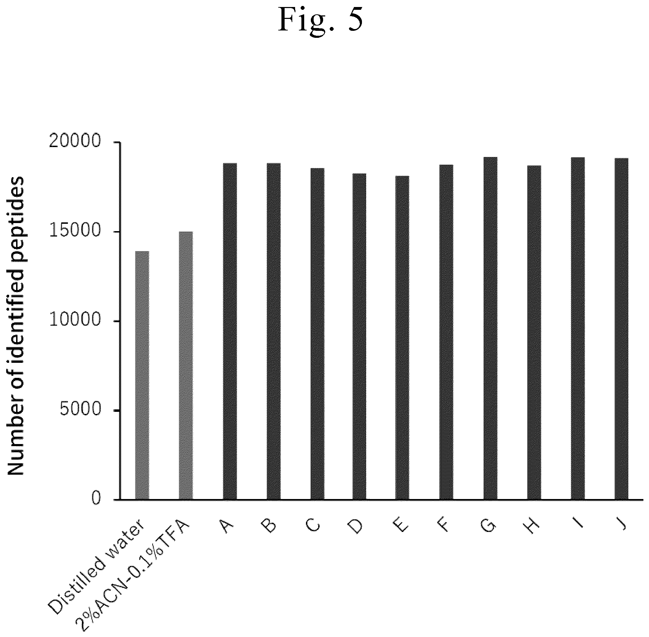

[Figure 5] Figure 5 shows the number of peptides that could be identified by LC-MS/MS. A-J on the horizontal axis corresponds to the sugar-based nonionic surfactants listed in Table 1.

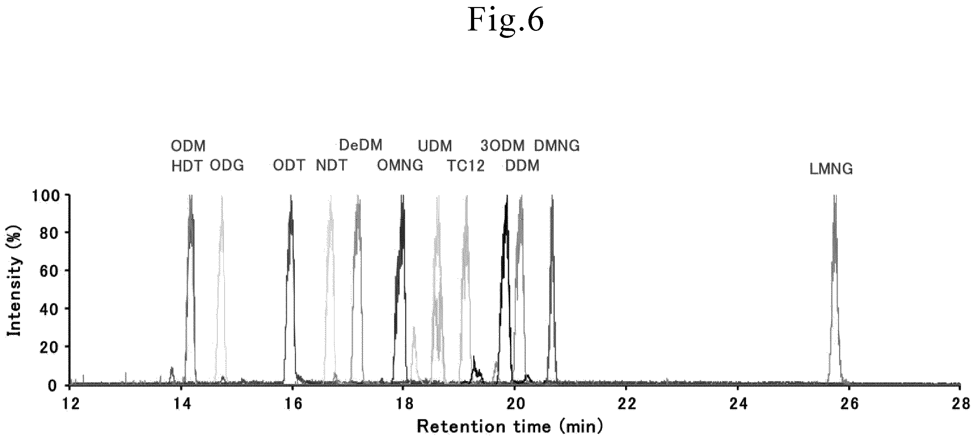

[Figure 6] Figure 6 shows the elution time of sugar-based surfactants in a reversed-phase (C18) column separation.

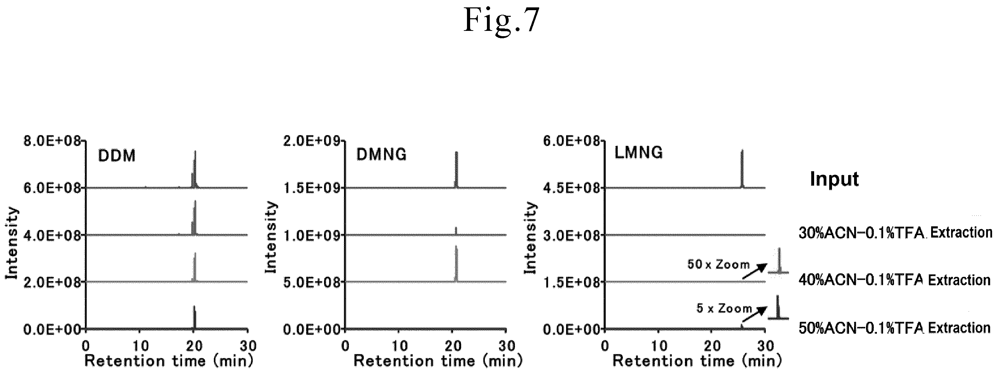

[Figure 7] Figure 7 shows the elution pattern when DDM, DMNG and LMNG were trapped in a reversed-phase (SDB) column and eluted continuously with increasing organic solvent concentration in the eluent.

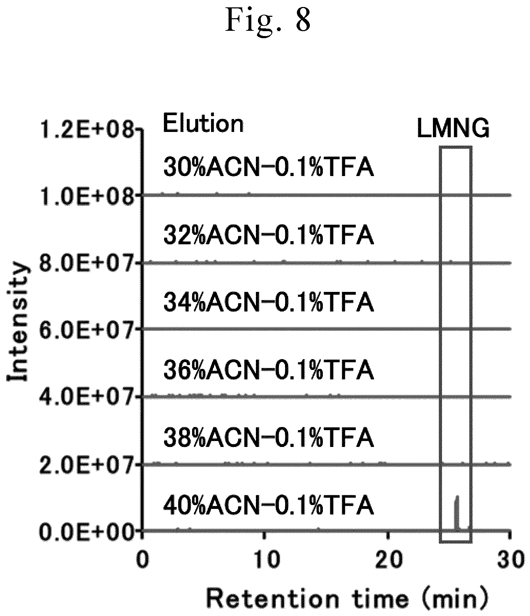

[Figure 8] Figure 8 shows evaluation of a detailed conditions of elution from a reversed-phase (SDB) column of LMNG.

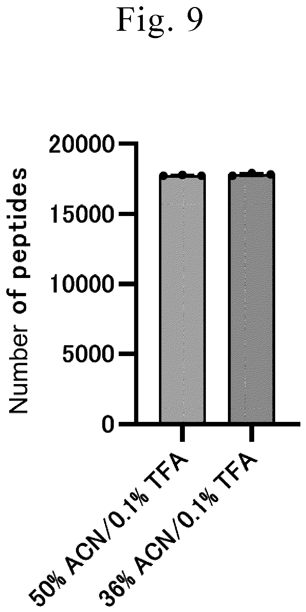

[Figure 9] Figure 9 shows that there is no change in the number of identified peptides between 50% ACN-0.1% TFA elution and 36% ACN-0.1% TFA elution.

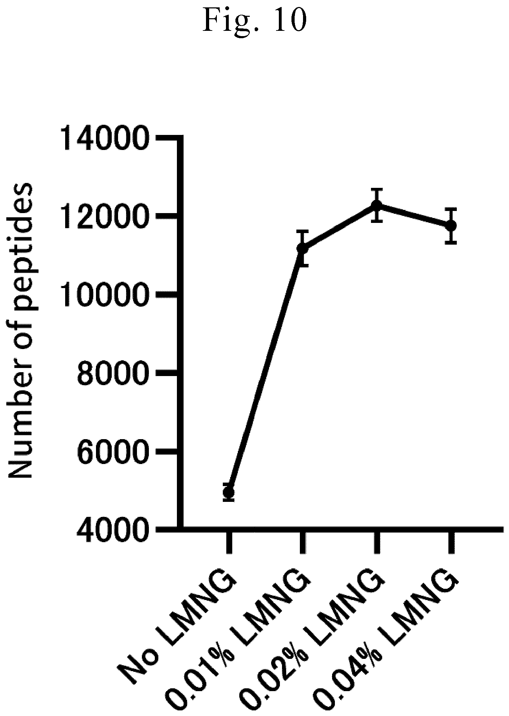

[Figure 10] Figure 10 is a graph showing that the addition of LMNG in enzymatic digestion of trace samples suppressed the loss of proteins and peptides during digestion.

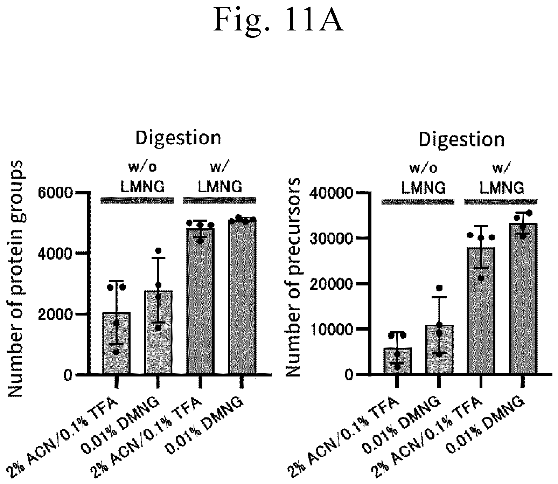

[Figure 11A] Figure 11A is the evaluation results showing that the addition of LMNG during enzymatic digestion and addition of DMNG during dissolution of dried peptide improves the number of identified proteins and precursors (peptides) in LC-MS.

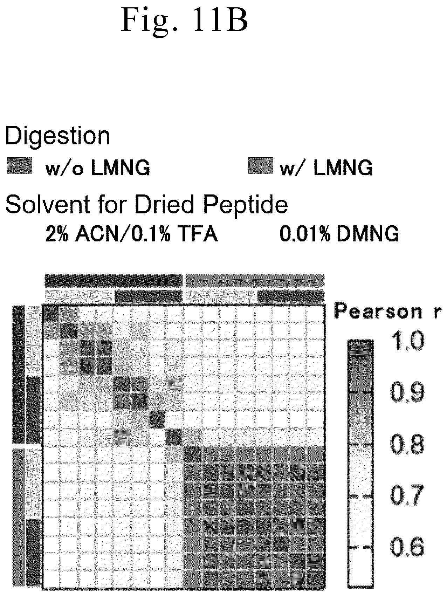

[Figure 11B] Figure 11B is the evaluation results showing that the addition of LMNG during enzymatic digestion and addition of DMNG during dissolution of dried peptide improves the reproducibility of LC-MS analysis.

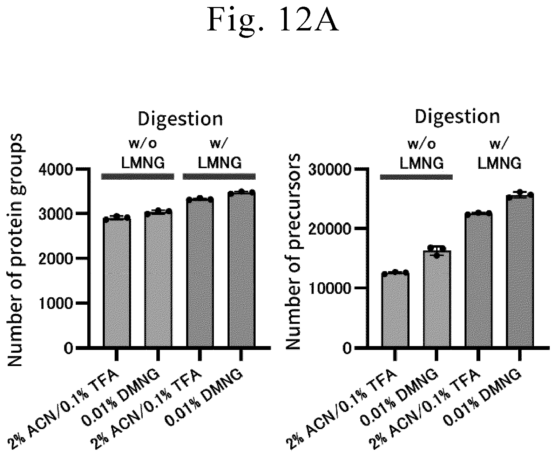

[Figure 12A] Figure 12A is the evaluation results showing that the addition of LMNG during enzymatic digestion and addition of DMNG during dissolution of dried peptide improves the number of identified proteins and precursors (peptides) in Co-IP-LC-MS.

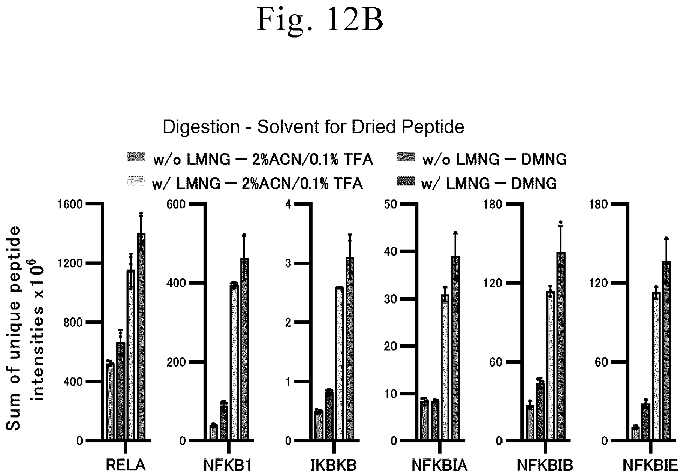

[Figure 12B] Figure 12B shows the total number of peptide fragments derived from RELA, NJKB1, IKBKB, NFKBIA, NFKBIB, and NFKBIE identified by Co-IP-LC-MS analysis with anti-RELA (NF-κB p65) antibody. The total number of peptide fragments increased with the addition of LMNG during enzymatic digestion and DMNG during dissolution of dried peptides.

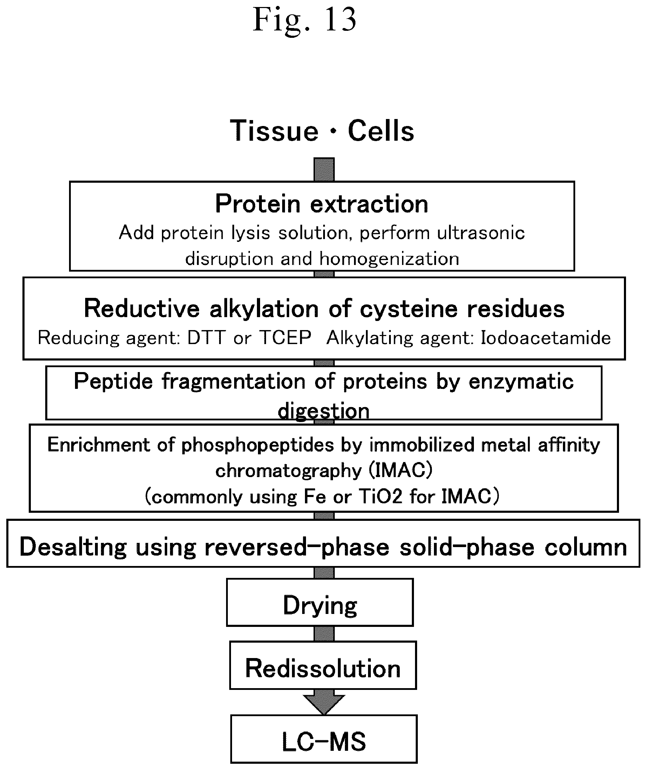

[Figure 13] Figure 13 shows the workflow of the pretreatment for the phosphoproteome analysis.

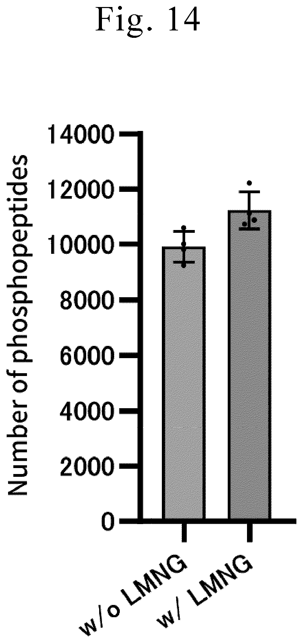

[Figure 14] Figure 14 is a graph showing that in immobilized metal affinity chromatography, the addition of LMNG to the eluent increased the recovery of phosphopeptides and resulted in higher number of identified phosphopeptides.

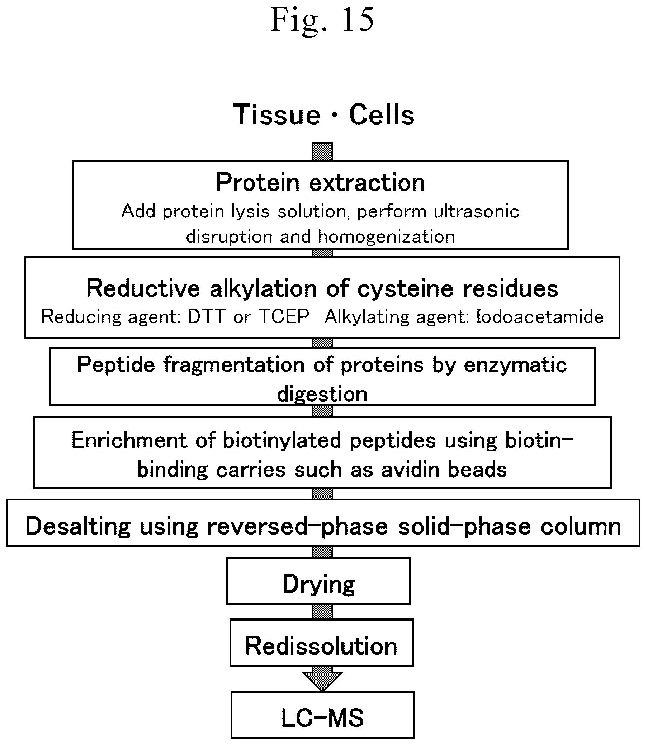

[Figure 15] Figure 15 shows the workflow of sample pretreatment for proteome analysis of biotinylated proteins.

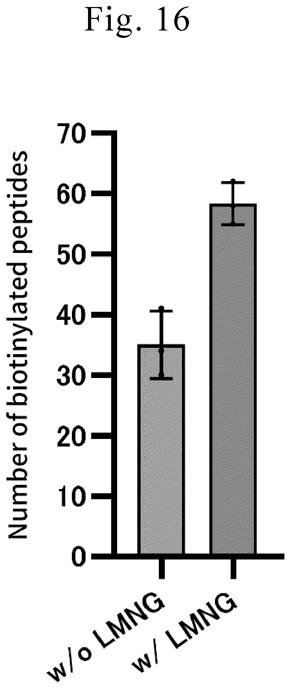

[Figure 16] Figure 16 is a graph showing that, in streptavidin affinity chromatography, the addition of LMNG to the eluent increased the recovery of biotinylated peptides and resulted in a higher number of identified biotinylated peptides.

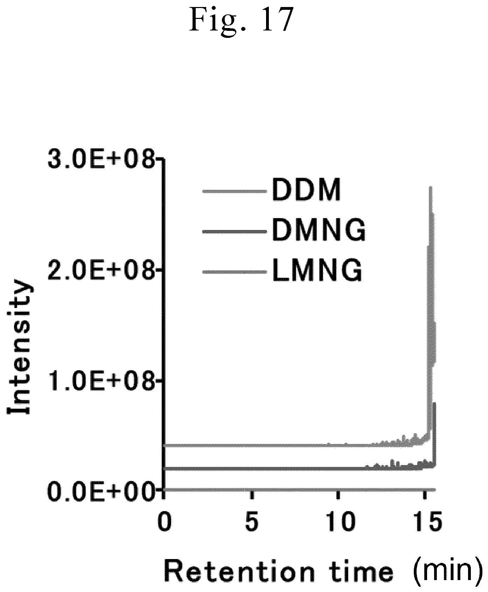

[Figure 17] Figure 17 shows the elution patterns of DDM, DMNG and LMNG in EVOSEP ONE.

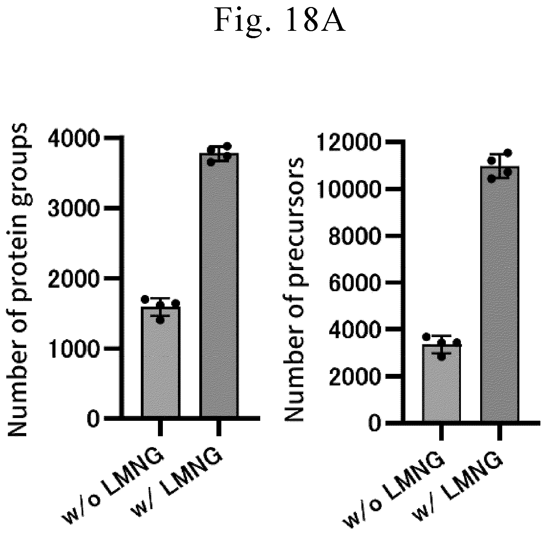

[Figure 18A] Figure 18A shows evaluation results indicating that the addition of LMNG during enzymatic digestion of HEK293 cell lysate improves the number of proteins and precursors (peptides) in LC-MS with EVOSEP ONE. To enable quantitative evaluation, data were acquired with DIA-MS.

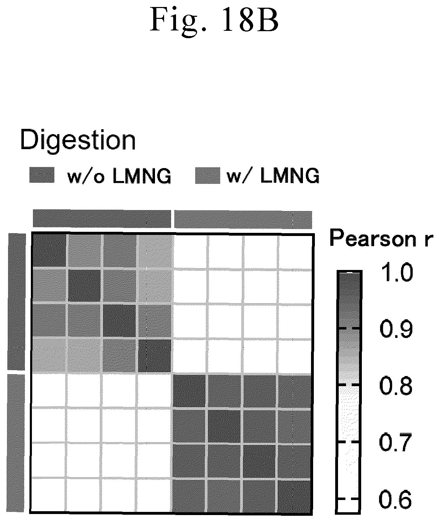

[Figure 18B] Figure 18B shows evaluation results indicating that the addition of LMNG during enzymatic digestion of cell lysate improved the reproducibility of LC-MS analysis results with EVOSEP ONE.

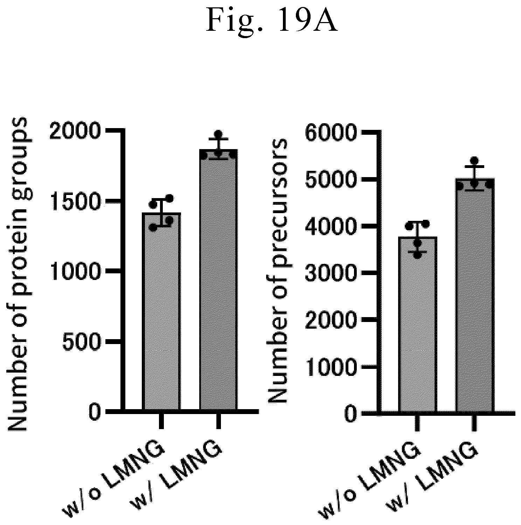

[Figure 19A] Figure 19A shows evaluation results indicating that the addition of LMNG during enzymatic digestion of serum exosomes improves the number of identified proteins and precursors (peptides) in LC-MS with EVOSEP ONE. To enable quantitative evaluation, data were acquired with DIA-MS.

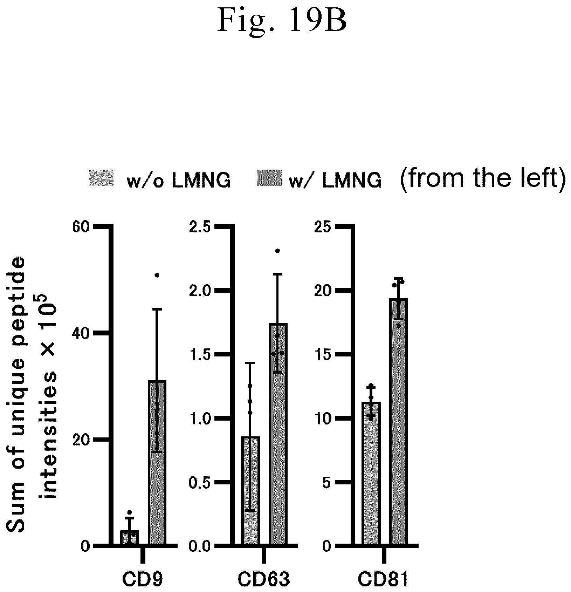

[Figure 19B] Figure 19B shows the evaluation results indicating that, in the proteome analysis of serum exosomes with EVOSEP ONE, the addition of LMNG during enzymatic digestion of serum exosomes improved the number of identified peptides derived from exosome-specific proteins CD9, CD63, and CD81. To enable quantitative evaluation, data were acquired with DIA-MS.

### MODE FOR CARRYING OUT THE INVENTION

### 1. Production Method of Aqueous Peptide Solution

The present invention provides a method for producing an aqueous peptide solution (hereinafter referred to as "the production method of the present invention"), which comprises dissolving a dried peptide in an aqueous solvent containing at least one sugar-based nonionic surfactant. In the production of an aqueous peptide solution, inclusion of at least one sugar-based nonionic surfactant in the aqueous solvent when dissolving the dried peptide accelerates the dissolution of the dried peptide that has been adsorbed onto the container, thereby avoiding undissolved peptide residues and also suppressing the re-adsorption of once-dissolved peptides to the vessel or the like. By adding and mixing an aqueous solvent containing at least one sugar-based nonionic surfactant to the dried peptide in a vessel, the dried peptide is dissolved in the aqueous solvent to obtain the intended aqueous peptide solution.

As used herein, the term "peptide" refers to a compound composed of a chain of amino acid residues linked by peptide bonds, and may be used interchangeably with the terms "polypeptide" and "protein." The length of the peptide used in the production method of the present invention is not particularly limited, but since sugar-based nonionic surfactants are excellent for accelerating the dissolution of long dried peptides, the peptide to be dissolved in the aqueous solvent comprises, at least in part, a long-chain peptide (for example, a peptide having a length of 11 or more amino acids, 12 or more amino acids, 13 or more amino acids, 14 or more amino acids, 15 or more amino acids, or 20 or more amino acids). The upper limit of the length of the long-chain peptide is not particularly limited, but is, for example, 100 amino acids or less, 80 amino acids or less, 60 amino acids or less, 50 amino acids or less, 40 amino acids or less, or 30 amino acids or less.

In the production method of the present invention, the peptide to be dissolved in an aqueous solvent may be a single type of peptide or a mixture of multiple types of peptides. In one embodiment, the peptide to be dissolved in the aqueous solvent in the production method of the present invention a mixture of multiple types of peptides and includes, at least in part, a long-chain peptide (for example, a peptide having a length of 11 or more amino acids, 12 or more amino acids, 13 or more amino acids, 14 or more amino acids, 15 or more amino acids, or 20 or more amino acids). The upper limit of the length of the long-chain peptide is not particularly limited, but is, for example, 100 amino acids or less, 80 amino acids or less, 60 amino acids or less, 50 amino acids or less, 40 amino acids or less, or 30 amino acids or less. The proportion (proportion of the molecular number) of long-chain peptides in the peptide mixture is not particularly limited, but may be, for example, 0.1% or more, 1% or more, 5% or more, 10% or more, 20% or more, 30% or more, 40% or more, or 50% or more.

In the production method of the present invention, the origin of the peptide to be dissolved in the aqueous solvent is not particularly limited, and it may be a peptide isolated from a biological material, chemically synthesized, or obtained as a recombinant product expressed by genetic engineering. In one embodiment, the peptide to be dissolved in the aqueous solvent in the production method of the present invention is a peptide mixture obtained by limited digestion of a biological sample containing a protein with a protease (e.g., trypsin).

In the production method of the present invention, a "dried" peptide is dissolved in an aqueous solvent. As used herein, "dried" means that the water content, chemically measured by at least one method selected from a drying method and the Karl Fischer method, is less than 15% (w/w) (e.g., less than 10% (w/w), less than 5% (w/w), or less than 1% (w/w)). Examples of methods for drying peptides include vacuum drying, heat drying, air drying, and freeze drying, but are not limited thereto.

In one embodiment, in the production method of the present invention, a dried peptide contained in a vessel having a plastic surface is dissolved in an aqueous solvent. The dissolution of the dried peptide adhered to the plastic surface inside the vessel may be accelerated by a sugar-based nonionic surfactant, and the re-adsorption of the peptide dissolved in the aqueous solvent onto the plastic surface may also be suppressed by the sugar-based nonionic surfactant. Examples of the type of plastic include, but are not limited to, polypropylene, polystyrene, polymethyl methacrylate, polyvinyl chloride, polyethylene, polycarbonate, polysulfone, fluoropolymers, polyamide, polydimethylsiloxane, polyurethane, polysulfone, polytetrafluoroethylene, and elastomers. The plastic surface inside the vessel may or may not be subjected to hydrophilic treatment. The "hydrophilic treatment" refers to a treatment for introducing hydrophilic groups such as hydroxyl groups or carboxyl groups onto the plastic surface of the vessel.

In one embodiment, in the production method of the present invention, a dried peptide adhered to the plastic surface inside a vessel is dissolved in an aqueous solvent. The dried peptide may be adhered to the plastic surface as a result of a drying process (e.g., vacuum drying).

The production method of the present invention is characterized in that at least one sugar-based nonionic surfactant is contained in an aqueous solvent used to dissolve a dried peptide. As used herein, a "sugar-based nonionic surfactant" refers to a nonionic surfactant having a sugar residue as a hydrophilic group unit. As used herein, a "sugar residue" refers to a monovalent group derived from a sugar structure, and specifically means a residue obtained by removing one hydroxyl group from a sugar. Preferably, the sugar residue is a residue obtained by removing one hydroxyl group bonded to the anomeric carbon at the reducing end of the sugar or one hydroxyl group in a hydroxymethyl group. The sugar-based nonionic surfactant used in the production method of the present invention is preferably a compound represented by formula (I);
[Chemistry 5]

G₁ - L₁ - R₁ (I)

(wherein G₁ is a monosaccharide or disaccharide residue, L₁ is O, S, -O-(CH₂)ₙ-O-, or -O-CO-, R₁ is a linear alkyl group having 6 to 12 carbon atoms, and n is an integer of 1 to 3), or by formula (II); 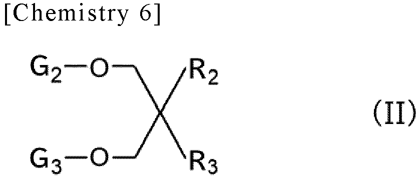 (wherein G₂ and G₃ are the same or different and represent a monosaccharide or disaccharide residue, and R₂ and R₃ are the same or different and represent a linear alkyl group having 6 to 12 carbon atoms).

As monosaccharides, pentoses or hexoses are preferred. Examples of pentoses include ribose, deoxyribose, fructose and the like. Examples of hexoses include glucose, mannose, galactose and the like. Examples of disaccharides include maltose, trehalose, lactose, isomaltose, sucrose, cellobiose and the like.

Examples of linear alkyl groups having 6 to 12 carbon atoms include hexyl group, heptyl group, octyl group, nonyl group, decyl group, undecyl group, and dodecyl group.

In formula (I), n is 1, 2 or 3, preferably 1 or 2, more preferably 2.

In formula (I), when L₁ is bonded to the anomeric carbon at the reducing end of G₁, the mode of the linkage may be either α-linkage or β-linkage.

In formula (I), G₁ is preferably a maltose residue, a trehalose residue, a glucose residue or a mannose residue.

In one embodiment of formula (I), G₁ is a maltose residue, a glucose residue, or a mannose residue, L₁ is O, S, or -O-(CH₂)ₙ-O-, R₁ is a linear alkyl group having 8 to 12 carbon atoms, n is 2, and L₁ is bonded to the anomeric carbon of G₁.

In one embodiment of formula (I), G₁ is a maltose residue or a glucose residue, L₁ is O or S, R₁ is a linear alkyl group having 8 to 12 carbon atoms, and L₁ is bonded to the anomeric carbon of G₁ via a β-linkage.

In another embodiment of formula (I), G₁ is a trehalose residue, L₁ is -O-CO-, and R₁ is a linear alkyl group having 8 to 12 carbon atoms.

Examples of compounds represented by formula (I) include:
n-dodecyl-β-D-maltoside (DDM),
n-undecyl-β-D-maltoside,
n-decyl-β-D-maltoside,
n-nonyl-β-D-thiomaltoside,
n-octyl-β-D-thioglucoside,
3-oxatridecyl-α-D-mannoside,
α-D-glucopyranosyl-α-D-glucopyranoside monododecanoate (trehalose C12),
and the like.

In formula (II), when oxygen atom is bonded to the anomeric carbon at the reducing end of G₂ or G₃, the mode of the bond may be either an α-linkage or a β-linkage.

In formula (II), G₂ and G₃ are preferably the same and are a glucose residue or a maltose residue.

In one embodiment of formula (II), G₂ and G₃ are the same and are a glucose residue or a maltose residue, R₂ and R₃ are the same and are a linear alkyl group having 6 to 10 carbon atoms, and the anomeric carbons at the reducing ends of G₂ and G₃ are bonded to the oxygen atom via a β-linkage.

Examples of compounds represented by formula (II) include: 2,2-dioctylpropane-1,3-bis-β-D-maltopyranoside (decyl maltose neopentyl glycol), 2,2-didecylpropane-1,3-bis-β-D-maltopyranoside (lauryl maltose neopentyl glycol), 2,2-dihexylpropane-1,3-bis-β-D-glucopyranoside (octylglucose neopentyl glycol), and the like.

The sugar-based nonionic surfactant is preferably selected from the following group:
n-dodecyl-β-D-maltoside (DDM),
n-undecyl-β-D-maltoside,
3-oxatridecyl-α-D-mannoside,
α-D-glucopyranosyl-α-D-glucopyranoside monododecanoate (trehalose C12),
2,2-dioctylpropane-1,3-bis-β-D-maltopyranoside (decyl maltose neopentyl glycol),
2,2-didecylpropane-1,3-bis-β-D-maltopyranoside (lauryl maltose neopentyl glycol), and
2,2-dihexylpropane-1,3-bis-β-D-glucopyranoside (octyl glucose neopentyl glycol).

In the production method of the present invention, one sugar-based nonionic surfactant may be included alone in the aqueous solvent, or two or more sugar-based nonionic surfactants may be used in combination and contained in the aqueous solvent. By combining two or more sugar-based nonionic surfactants, an enhanced effect in accelerating the dissolution of a dried peptide may be expected compared to the use of a single sugar-based nonionic surfactant alone. In one embodiment, the aqueous solvent used in the production method of the present invention contains a combination of at least two sugar-based nonionic surfactants.

The species of sugar-based nonionic surfactants to be combined are not particularly limited, but are preferably a combination of at least two compounds selected from among the compounds represented by the above-mentioned formula (I) and the compounds represented by the formula (II).

In one embodiment, the aqueous solvent used in the production method of the present invention contains a combination of at least two sugar-based nonionic surfactants selected from the following group:
n-dodecyl-β-D-maltoside (DDM);
3-oxatridecyl-α-D-mannoside;
α-D-glucopyranosyl-α-D-glucopyranoside monododecanoate (trehalose C12);
2,2-dioctylpropane-1,3-bis-β-D-maltopyranoside (decyl maltose neopentyl glycol);
2,2-didecylpropane-1,3-bis-β-D-maltopyranoside (lauryl maltose neopentyl glycol); and
2,2-dihexylpropane-1,3-bis-β-D-glucopyranoside (octyl glucose neopentyl glycol).

Preferred combinations of sugar-based nonionic surfactants include the followings:
n-dodecyl-β-D-maltoside and α-D-glucopyranosyl-α-D-glucopyranoside monodododecanoate;
2,2-dihexylpropane-1,3-bis-β-D-glucopyranoside and α-D-glucopyranosyl-α-D-glucopyranoside monodododecanoate;
2,2-dioctylpropane-1,3-bis-β-D-maltopyranoside and 2,2-dihexylpropane-1,3-bis-β-D-glucopyranoside; and
3-oxatridecyl-α-D-mannoside and 2,2-dioctylpropane-1,3-bis-β-D-maltopyranoside.

The aqueous solvent may further contain additional components other than water and the sugar-based nonionic surfactant. For example, the aqueous solvent may contain surfactants other than the sugar-based nonionic surfactant, a buffering agent, or a hydrophilic organic solvent, or the like. When producing an aqueous peptide solution for analysis using LC-MS or the like, it is preferable to avoid containing components that may interfere with the analysis or contaminate the analytical instrument. In one embodiment, the aqueous solvent consists of water and a sugar-based nonionic surfactant.

The total concentration of sugar-based nonionic surfactants contained in the aqueous solvent (i.e., the sum of the concentrations of all species of sugar-based nonionic surfactants contained in the aqueous solvent when two or more sugar-based nonionic surfactants are contained in the aqueous solvent) may be appropriately adjusted in consideration of factors such as the species of sugar-based nonionic surfactants and the amount of dried peptide to be dissolved. For example, the concentration may be 0.00125% (w/w) or more, 0.0025% (w/w) or more, 0.005% (w/w) or more, or 0.01% (w/w) or more.

Table 1 shows examples of the minimum concentrations of sugar-based nonionic surfactants in the aqueous solvent when one sugar-based nonionic surfactant is used alone.

**[Table 1]**

| | Sugar-Based Nonionic Surfactant | Minimum Concentration (w/w%) |
|---|---|---|
| A | n-dodecyl-β-D-maltoside (DDM) | 0.0025 |
| B | n-undecyl-β-D-maltoside | 0.0025 |
| C | n-decyl-β-D-maltoside | 0.0025 |
| D | n-nonyl-β-D-thiomaltoside | 0.005 |
| E | n-octyl-β-D-thioglucoside | 0.01 |
| F | 3-oxatridecyl-α-D-mannoside | 0.00125 |
| G | 2,2-dioctylpropane-1,3-bis-β-D-maltopyranoside (Decyl maltose neopentyl glycol) | 0.0025 |
| H | 2,2-didodecylpropane-1,3-bis-β-D-maltopyranoside (Lauryl maltose neopentyl glycol) | 0.00125 |
| I | 2,2-dihexylpropane-1,3-bis-β-D-glucopyranoside (Octyl glucose neopentyl glycol) | 0.00125 |
| J | α-D-glucopyranosyl-α-D-glucopyranoside monododecanoate (Trehalose C12) | 0.0025 |

When each sugar-based nonionic surfactant is used alone, it is preferable that the sugar-based nonionic surfactant be contained in the aqueous solvent at a concentration equal to or higher than the minimum concentration listed in Table 1, from the viewpoint of ensuring accelerated dissolution of the dried peptide.

By combining two or more sugar-based nonionic surfactants, an enhanced effect on accelerating the dissolution of dried peptides can be expected. Therefore, even if the concentration of each sugar-based nonionic surfactant in the aqueous solvent is lower than the minimum concentration listed in Table 1, a certain degree of accelerated dissolution of the dried peptide may still be expected when two or more sugar-based nonionic surfactants are used in combination. However, in order to more reliably accelerate the dissolution of the dried peptide, it is preferable to adjust the concentration of each sugar-based nonionic surfactant in the aqueous solvent to be equal to or higher than the minimum concentration listed in Table 1 when two or more sugar-based nonionic surfactants are used in combination.

In one embodiment, when two or more sugar-based nonionic surfactants are used in combination, the concentration of each sugar-based nonionic surfactant in the aqueous solvent is adjusted to 0.005% (w/w) or more.

Although it is not possible to unambiguously define an upper limit for the total concentration of sugar-based nonionic surfactants contained in the aqueous solvent, the acceleration of dried peptide dissolution does not require micelle formation by the sugar-based nonionic surfactants. Therefore, even when the concentration of the sugar-based nonionic surfactant is below its critical micelle concentration (CMC), the dissolution of the dried peptide may be accelerated. In one embodiment, the total concentration of sugar-based nonionic surfactants contained in the aqueous solvent is below the CMC of the added sugar-based nonionic surfactants. When producing an aqueous peptide solution for analysis by LC-MS or the like, it is preferable that the concentration of the sugar-based nonionic surfactant be as low as possible within a range that still provides a sufficient dissolution-accelerating effect, in order to avoid contamination of the analytical instrument. In one embodiment, the total concentration of sugar-based nonionic surfactants contained in the aqueous solvent is 0.04% (w/w) or less.

2. Agent for Accelerating Dissolution of a Dried Peptide The present invention provides an agent for accelerating dissolution of a dried peptide (hereinafter referred to as "the agent for accelerating dissolution of a dried peptide of the present invention"), which comprises at least one sugar-based nonionic surfactant. In the above-described production method of the present invention, an aqueous peptide solution can be easily produced by adding the agent for accelerating dissolution of dried peptides of the present invention to the aqueous solvent. The agent for accelerating dissolution of dried peptides of the present invention may also serve as an aqueous solvent for dissolving a dried peptide.

The definition of "sugar-based nonionic surfactant" is the same as the definition described in Section 1 above. The sugar-based nonionic surfactant used in the agent for accelerating dissolution of a dried peptide of the present invention is preferably a compound represented by formula (I);
[Chemistry 7]

G₁ - L₁ - R₁ (I)

or formula (II); 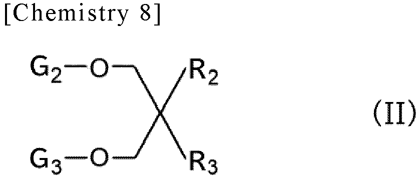 (wherein the definitions of the respective substituents in formulae (I) and (II) are the same as those described in Section 1 above.)

Examples of compounds represented by formula (I) include:
n-dodecyl-β-D-maltoside (DDM),
n-undecyl-β-D-maltoside,
n-decyl-β-D-maltoside,
n-nonyl-β-D-thiomaltoside,
n-octyl-β-D-thioglucoside,
3-oxatridecyl-α-D-mannoside,
α-D-glucopyranosyl-α-D-glucopyranoside monododecanoate (trehalose C12),
and the like.

Examples of compounds represented by formula (II) include:
2,2-dioctylpropane-1,3-bis-β-D-maltopyranoside (decyl maltose neopentyl glycol),
2,2-didecylpropane-1,3-bis-β-D-maltopyranoside (lauryl maltose neopentyl glycol),
2,2-dihexylpropane-1,3-bis-β-D-glucopyranoside (octylglucose neopentyl glycol),
and the like.

The sugar-based nonionic surfactant is preferably selected from the following group:
n-dodecyl-β-D-maltoside (DDM),
n-undecyl-β-D-maltoside,
3-oxatridecyl-α-D-mannoside,
α-D-glucopyranosyl-α-D-glucopyranoside monododecanoate (trehalose C12),
2,2-dioctylpropane-1,3-bis-β-D-maltopyranoside (decyl maltose neopentyl glycol),
2,2-didecylpropane-1,3-bis-β-D-maltopyranoside (lauryl maltose neopentyl glycol), and
2,2-dihexylpropane-1,3-bis-β-D-glucopyranoside (octyl glucose neopentyl glycol).

The agent for accelerating dissolution of a dried peptide of the present invention may contain one sugar-based nonionic surfactant alone, or may contain a combination of two or more sugar-based nonionic surfactants. In one embodiment, the agent for accelerating dissolution of a dried peptide of the present invention contains a combination of at least two sugar-based nonionic surfactants.

The species of sugar-based nonionic surfactants to be combined are not particularly limited, but are preferably a combination of at least two species selected from among the compound represented by the above-mentioned formula (I) and the compound represented by formula (II).

In one embodiment, the agent for accelerating dissolution of a dried peptide of the present invention contains a combination of at least two sugar-based nonionic surfactants selected from the following group:
n-dodecyl-β-D-maltoside (DDM),
3-oxatridecyl-α-D-mannoside,
α-D-glucopyranosyl-α-D-glucopyranoside monodododecanoate (trehalose C12),
2,2-dioctylpropane-1,3-bis-β-D-maltopyranoside (decyl maltose neopentyl glycol),
2,2-didecylpropane-1,3-bis-β-D-maltopyranoside (lauryl maltose neopentyl glycol), and
2,2-dihexylpropane-1,3-bis-β-D-glucopyranoside (octylglucose neopentyl glycol).

Preferred combinations of sugar-based nonionic surfactants include the followings:
n-dodecyl-β-D-maltoside and α-D-glucopyranosyl-α-D-glucopyranoside monodododecanoate;
2,2-dihexylpropane-1,3-bis-β-D-glucopyranoside and α-D-glucopyranosyl-α-D-glucopyranoside monodododecanoate;
2,2-dioctylpropane-1,3-bis-β-D-maltopyranoside and 2,2- dihexylpropane-1,3-bis-β-D-glucopyranoside; and
3-oxatridecyl-α-D-mannoside and 2,2-dioctylpropane-1,3-bis-β-D-maltopyranoside.

The agent for accelerating dissolution of a dried peptide of the present invention may be provided in the form of a powder of a sugar-based nonionic surfactant (e.g., a freeze-dried product) or in the form of an aqueous solvent containing a sugar-based nonionic surfactant (e.g., a solution of a sugar-based nonionic surfactant in an aqueous solvent, a suspension of a sugar-based nonionic surfactant in an aqueous solvent, or the like).

The aqueous solvent may further contain additional components other than water and the sugar-based nonionic surfactant. For example, the aqueous solvent may contain surfactants other than the sugar-based nonionic surfactant, a buffering agent, or a hydrophilic organic solvent, or the like. When producing an aqueous peptide solution for analysis using LC-MS or the like, it is preferable to avoid containing components that may interfere with the analysis or contaminate the analytical instrument. In one embodiment, the aqueous solvent consists of water and a sugar-based nonionic surfactant.

In one embodiment, the agent for accelerating dissolution of a dried peptide of the present invention is an aqueous solvent containing a sugar-based nonionic surfactant, which can be used as it is, without dilution, as the aqueous solvent for dissolving a dried peptide in the above-mentioned production method of the present invention (non-concentrated type).

In the non-concentrated type, the total concentration of sugar-based nonionic surfactants contained in the aqueous solvent may be appropriately adjusted in consideration of the species of the sugar-based nonionic surfactant, the amount of dried peptide to be dissolved and the like. For example, the concentration may be 0.00125% (w/w) or more, 0.0025% (w/w) or more, 0.005% (w/w) or more, or 0.01% (w/w) or more.

Examples of the minimum concentrations of sugar-based nonionic surfactants in the aqueous solvent when one sugar-based nonionic surfactant is used alone in the non-concentrated type are shown in Table 1 above.

When each sugar-based nonionic surfactant is used alone, it is preferable that the sugar-based nonionic surfactant be contained in the aqueous solvent at a concentration equal to or higher than the minimum concentration listed in Table 1, from the viewpoint of ensuring accelerated dissolution of the dried peptide.

By combining two or more sugar-based nonionic surfactants, an enhanced effect on accelerating the dissolution of dried peptides can be expected. Therefore, even if the concentration of each sugar-based nonionic surfactant in the aqueous solvent is lower than the minimum concentration listed in Table 1, a good accelerated dissolution of the dried peptide may still be expected when two or more sugar-based nonionic surfactants are used in combination. However, in order to more reliably accelerate the dissolution of the dried peptide, in the embodiment of the non-concentrated type, it is preferable to adjust the concentration of each sugar-based nonionic surfactant in the aqueous solvent to be equal to or higher than the minimum concentration listed in Table 1 when two or more sugar-based nonionic surfactants are used in combination.

In one embodiment of the non-concentrated type, two or more sugar-based nonionic surfactants are combined, and the concentration of each sugar-based nonionic surfactant in the aqueous solvent is adjusted to 0.005% (w/w) or more.

In the non-concentrated type, although it is not possible to unambiguously define an upper limit for the total concentration of sugar-based nonionic surfactants contained in the aqueous solvent, the acceleration of dried peptide dissolution does not require micelle formation by the sugar-based nonionic surfactants. Therefore, even when the concentration of the sugar-based nonionic surfactant is below its critical micelle concentration (CMC), the dissolution of the dried peptide may be accelerated. In one embodiment, the total concentration of sugar-based nonionic surfactants contained in the aqueous solvent is below the CMC of the added sugar-based nonionic surfactants. When producing an aqueous peptide solution for analysis by LC-MS or the like, it is preferable that the concentration of the sugar-based nonionic surfactant be as low as possible within a range that still provides a sufficient dissolution-accelerating effect, in order to avoid contamination of the analytical instrument. In one embodiment, the total concentration of sugar-based nonionic surfactants contained in the aqueous solvent is 0.04% (w/w) or less.

In one embodiment, the agent for accelerating dissolution of dried peptides of the present invention is an aqueous solvent containing a sugar-based nonionic surfactant, and an aqueous solvent for dissolving a dried peptide can be obtained by diluting said agent with an appropriate aqueous solvent (e.g., water) at an appropriate dilution ratio (concentrated type). The dilution ratio is not particularly limited, but is, for example, 2-fold to 100-fold (e.g., ×2, ×5, ×10, ×20, or ×100) in consideration of the convenience of the experimenter.

In the concentrated type, although the total concentration of sugar-based nonionic surfactants contained in the aqueous solvent may be appropriately adjusted in consideration of the species of the sugar-based nonionic surfactant, the amount of dried peptide to be dissolved and the like, the total concentration of sugar-based nonionic surfactants, when diluted 2-fold to 100-fold (e.g., ×2, ×5, ×10, ×20, or ×100) with an aqueous solvent (e.g., water), is, for example, 0.00125% (w/w) or more, 0.0025% (w/w) or more, 0.005% (w/w) or more, or 0.01% (w/w) or more.

In the concentrated type, when one sugar-based nonionic surfactant is used alone, examples of the minimum concentrations of the sugar-based nonionic surfactant in the aqueous solvent after diluting the agent for accelerating dissolution of a dried peptide of the present invention 2-fold to 100-fold (e.g., ×2, ×5, ×10, ×20, or ×100) with an aqueous solvent are shown in Table 1 above.

In the concentrated type, when each sugar-based nonionic surfactant listed in Table 1 is used alone, it is preferable to prepare the agent for accelerating dissolution of a dried peptide of the present invention so that, after 2-fold to 100-fold (e.g., ×2, ×5, ×10, ×20, or ×100) dilution with an aqueous solvent (e.g., water), the concentration of each sugar-based nonionic surfactant becomes equal to or higher than the minimum concentration listed in Table 1, from the viewpoint of ensuring the acceleration of dissolution of dried peptides.

When combining two or more sugar-based nonionic surfactants in the concentrated type agent for accelerating dissolution of a dried peptide of the present invention, good acceleration of dissolution of a dried peptide can be expected even if the concentration of each sugar-based nonionic surfactant after dilution with an aqueous solvent at an appropriate dilution ratio (e.g., 2-fold to 100-fold (e.g., ×2, ×5, ×10, ×20, or ×100)) is lower than the minimum concentration listed in Table 1. However, in order to more reliably accelerate the dissolution of a dried peptide, it is preferable to prepare the agent for accelerating dissolution of a dried peptide of the present invention so that, after dilution with an aqueous solvent at an appropriate dilution ratio (e.g., 2-fold to 100-fold (e.g., ×2, ×5, ×10, ×20, or ×100)), the concentration of each sugar-based nonionic surfactant in the aqueous solvent becomes equal to or higher than the minimum concentration listed in Table 1.

In one embodiment of the concentrated type, when two or more species of sugar-based nonionic surfactants are combined, the agent for accelerating dissolution of a dried peptide of the present invention is prepared so that, after 2-fold to 100-fold (e.g., ×2, ×5, ×10, ×20, or ×100) dilution with an aqueous solvent, the concentration of each sugar-based nonionic surfactant in the aqueous solvent becomes 0.005% (w/w) or more.

Regarding the concentrated type, it is not possible to unambiguously define an upper limit for the total concentration of sugar-based nonionic surfactants contained in the aqueous solvent. In one embodiment, the final total concentration of sugar-based nonionic surfactants after dilution with an aqueous solvent at an appropriate dilution ratio (e.g., 2-fold to 100-fold (e.g., ×2, ×5, ×10, ×20, or ×100)) is below the critical micelle concentration (CMC) of the added sugar-based nonionic surfactant(s). When producing an aqueous peptide solution for analysis by LC-MS or the like, it is preferable that the concentration of the sugar-based nonionic surfactant be as low as possible within a range that still provides a sufficient dissolution-accelerating effect, in order to avoid contamination of the analytical instrument. In one embodiment, the agent for accelerating dissolution of a dried peptide of the present invention is prepared so that the final total concentration of sugar-based nonionic surfactants after dilution with an aqueous solvent at an appropriate dilution ratio (e.g., 2-fold to 100-fold (e.g., ×2, ×5, ×10, ×20, or × 100)) is 0.04% (w/w) or less.

Unless otherwise specified, the definitions of the respective terms regarding the agent for accelerating dissolution of the present invention are the same as those described for the production method of the present invention.

### 3. Aqueous Peptide Solution

The present invention provides an aqueous peptide solution (hereinafter referred to as "the aqueous solution of the present invention") comprising a peptide and at least one sugar-based nonionic surfactant. The aqueous solution of the present invention is stable since adsorption of the peptide to a vessel, etc. is suppressed by containing at least one sugar-based nonionic surfactant. The aqueous solution of the present invention can be obtained by dissolving a dried peptide in an aqueous solvent containing at least one sugar-based nonionic surfactant according to the above-mentioned production method of the present invention.

The length of the peptide contained in the aqueous solution of the present invention is not particularly limited. However, since the sugar-based nonionic surfactant is excellent in suppressing the adsorption of long peptides to a vessel, the peptide contained in the aqueous solution of the present invention includes, at least in part, a long-chain peptide (for example, a peptide having a length of 11 amino acids or more, 12 amino acids or more, 13 amino acids or more, 14 amino acids or more, 15 amino acids or more, or 20 amino acids or more). The upper limit of the length of the long-chain peptide is not particularly limited, but may be, for example, 100 amino acids or less, 80 amino acids or less, 60 amino acids or less, 50 amino acids or less, 40 amino acids or less, or 30 amino acids or less.

The peptide contained in the aqueous solution of the present invention may be a single species of peptide or a mixture of multiple species of peptides. In one embodiment, the peptide contained in the aqueous solution of the present invention is a mixture of multiple species of peptides and includes, at least in part, a long-chain peptide (for example, a peptide having a length of 11 amino acids or more, 12 amino acids or more, 13 amino acids or more, 14 amino acids or more, 15 amino acids or more, or 20 amino acids or more). The upper limit of the length of the long-chain peptide is not particularly limited, but may be, for example, 100 amino acids or less, 80 amino acids or less, 60 amino acids or less, 50 amino acids or less, 40 amino acids or less, or 30 amino acids or less. The proportion (proportion of the molecular number) of long-chain peptides in the peptide mixture is not particularly limited, but may be, for example, 0.1% or more, 1% or more, 5% or more, 10% or more, 20% or more, 30% or more, 40% or more, or 50% or more.

In one embodiment, the proportion (proportion of the molecular number) of peptides having a length of 100 amino acids or less (for example, 80 amino acids or less, 60 amino acids or less, 50 amino acids or less, 40 amino acids or less, or 30 amino acids or less) among the peptides contained in the aqueous solution of the present invention may be, for example, 50% or more, 60% or more, 70% or more, 80% or more, or 90% or more.

The origin of the peptide contained in the aqueous solution of the present invention is not particularly limited, and it may be one isolated from a biological material, chemically synthesized, or obtained as a recombinant product expressed by genetic engineering techniques. In one embodiment, the peptides contained in the aqueous solution of the present invention is a mixture of peptides obtained by limited digestion of a protein derived from a biological material using a protease (for example, trypsin).

The concentration of the peptide contained in the aqueous solution of the present invention is not particularly limited, but may be, for example, 1 fg/mL or more, 10 fg/mL or more, 100 fg/mL or more, 1 pg/mL or more, 10 pg/mL or more, or 100 pg/mL or more. In the aqueous solution of the present invention, the peptide is contained at a concentration not exceeding its solubility, which may be, for example, 100 mg/mL or less, 10 mg/mL or less, 1 mg/mL or less, or 100 µg/mL or less. In low-concentration aqueous peptide solutions, loss due to adsorption of peptides to vessels or the like is a problem; however, since the aqueous solution of the present invention contains at least one sugar-based nonionic surfactant, such adsorption is suppressed, thereby enabling the provision of a stable low-concentration aqueous peptide solution. In one embodiment, the concentration of the peptide contained in the aqueous solution of the present invention is, for example, 100 µg/mL or less, 10 µg/mL or less, 1 µg/mL or less, 100 ng/mL or less, 10 ng/mL or less, or 1 ng/mL or less.

The aqueous solution of the present invention is characterized in that it contains at least one sugar-based nonionic surfactant. The definition of "sugar-based nonionic surfactant" is the same as that described in Section 1 above. The sugar-based nonionic surfactant contained in the aqueous solution of the present invention is preferably a compound represented by formula (I);
[Chemistry 9]

G₁ - L₁ - R₁ (I)

or by formula (II); 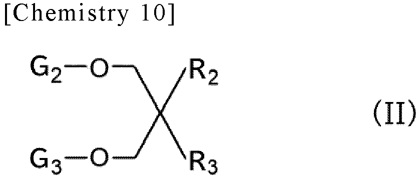 (wherein the definitions of the respective substituents in formulae (I) and (II) are the same as those described in Section 1 above.)

Examples of compounds represented by formula (I) include:
n-dodecyl-β-D-maltoside (DDM),
n-undecyl-β-D-maltoside,
n-decyl-β-D-maltoside,
n-nonyl-β-D-thiomaltoside,
n-octyl-β-D-thioglucoside,
3-oxatridecyl-α-D-mannoside,
α-D-glucopyranosyl-α-D-glucopyranoside monododecanoate (trehalose C12),
and the like.

Examples of compounds represented by formula (II) include:
2,2-dioctylpropane-1,3-bis-β-D-maltopyranoside (decyl maltose neopentyl glycol),
2,2-didecylpropane-1,3-bis-β-D-maltopyranoside (lauryl maltose neopentyl glycol),
2,2-dihexylpropane-1,3-bis-β-D-glucopyranoside (octylglucose neopentyl glycol),
and the like.

The sugar-based nonionic surfactant is preferably selected from the following group:
n-dodecyl-β-D-maltoside (DDM),
n-undecyl-β-D-maltoside,
3-oxatridecyl-α-D-mannoside,
α-D-glucopyranosyl-α-D-glucopyranoside monododecanoate (trehalose C12),
2,2-dioctylpropane-1,3-bis-β-D-maltopyranoside (decyl maltose neopentyl glycol),
2,2-didecylpropane-1,3-bis-β-D-maltopyranoside (lauryl maltose neopentyl glycol), and
2,2-dihexylpropane-1,3-bis-β-D-glucopyranoside (octyl glucose neopentyl glycol).

The aqueous solution of the present invention may contain one sugar-based nonionic surfactant alone, or may contain a combination of two or more sugar-based nonionic surfactants. In one embodiment, the aqueous solution of the present invention contains a combination of at least two sugar-based nonionic surfactants.

The species of sugar-based nonionic surfactants to be combined are not particularly limited, but preferably at least two compounds selected from among the compound represented by the above-mentioned formula (I) and the compound represented by formula (II) are combined.

In one embodiment, the aqueous solution of the present invention contains a combination of at least two sugar-based nonionic surfactants selected from the following group:
n-dodecyl-β-D-maltoside (DDM),
3-oxatridecyl-α-D-mannoside,
α-D-glucopyranosyl-α-D-glucopyranoside monodododecanoate (trehalose C12),
2,2-dioctylpropane-1,3-bis-β-D-maltopyranoside (decyl maltose neopentyl glycol),
2,2-didecylpropane-1,3-bis-β-D-maltopyranoside (lauryl maltose neopentyl glycol), and
2,2-dihexylpropane-1,3-bis-β-D-glucopyranoside (octylglucose neopentyl glycol).

Preferred combinations of sugar-based nonionic surfactants include the followings:
n-dodecyl-β-D-maltoside and α-D-glucopyranosyl-α-D-glucopyranoside monodododecanoate;
2,2-dihexylpropane-1,3-bis-β-D-glucopyranoside and α-D-glucopyranosyl-α-D-glucopyranoside monodododecanoate;
2,2-dioctylpropane-1,3-bis-β-D-maltopyranoside and 2,2- dihexylpropane-1,3-bis-β-D-glucopyranoside; and
3-oxatridecyl-α-D-mannoside and 2,2-dioctylpropane-1,3-bis-β-D-maltopyranoside.

In a further aspect, the aqueous solution of the present invention contains, as the surfactant, only 2,2-didecylpropane-1,3-bis-β-D-maltopyranoside alone.

The aqueous solution of the present invention may further contain additional components other than the peptide, sugar-based nonionic surfactant and water. For example, the aqueous solution of the present invention may contain surfactants other than the sugar-based nonionic surfactant, a buffering agent, or a hydrophilic organic solvent, or the like. When the aqueous solution of the present invention is used for analysis using LC-MS or the like, it is preferable to avoid containing components that may interfere with the analysis or contaminate the analytical instrument. In one embodiment, the aqueous solution of the present invention consists of a peptide, sugar-based nonionic surfactant, and water.

The total concentration of sugar-based nonionic surfactants contained in the aqueous solution of the present invention (i.e., the sum of the concentrations of all species of sugar-based nonionic surfactants contained in the aqueous solution of the present invention when two or more sugar-based nonionic surfactants are contained in said solution) may be appropriately adjusted in consideration of factors such as the species of sugar-based nonionic surfactants and the amount of dried peptide to be dissolved. For example, the concentration may be 0.00125% (w/w) or more, 0.0025% (w/w) or more, 0.005% (w/w) or more, or 0.01% (w/w) or more.

Table 1 above shows examples of the minimum concentration of the sugar-based nonionic surfactant in the aqueous solvent when the aqueous solution of the present invention contains one sugar-based nonionic surfactant alone. When the aqueous solution of the present invention contains each sugar-based nonionic surfactant alone, it is preferable that the aqueous solution contains each sugar-based nonionic surfactant at a concentration equal to or higher than the minimum concentration listed in Table 1, from the viewpoint of ensuring the effect of suppressing peptide adsorption onto a vessel.

By combining two or more sugar-based nonionic surfactants, an enhanced effect of suppressing peptide adsorption onto a vessel can be expected. Therefore, even if the concentration of each sugar-based nonionic surfactant in the aqueous solution is lower than the minimum concentration listed in Table 1 when two or more sugar-based nonionic surfactants are used in combination, a certain peptide adsorption-suppressing effect can still be expected. However, in order to reliably suppress peptide adsorption, even when two or more sugar-based nonionic surfactants are combined, it is preferable to adjust the concentration of each sugar-based nonionic surfactant in the aqueous solution to be equal to or higher than the minimum concentration listed in Table 1.

In one embodiment, when two or more sugar-based nonionic surfactants are combined, the concentration of each sugar-based nonionic surfactant in the aqueous solution of the present invention is adjusted to 0.005% (w/w) or more.

Although it is not possible to unambiguously define an upper limit for the total concentration of sugar-based nonionic surfactants contained in the aqueous solution of the present invention, the suppression of peptide adsorption onto a vessel does not require micelle formation by the sugar-based nonionic surfactants. Therefore, even when the concentration of the sugar-based nonionic surfactant is below its critical micelle concentration (CMC), the peptide adsorption can be suppressed. In one embodiment, the total concentration of sugar-based nonionic surfactants contained in the aqueous solution of the present invention is below the CMC of the added sugar-based nonionic surfactants. When the aqueous solution of the present invention is used for analysis by LC-MS or the like, it is preferable that the concentration of the sugar-based nonionic surfactant be as low as possible in order to avoid contamination of the analytical instrument. In one embodiment, the total concentration of sugar-based nonionic surfactants contained in the aqueous solution of the present invention is 0.04% (w/w) or less.

In one embodiment, the aqueous solution of the present invention is provided as a preparation contained in a vessel having a plastic surface. Since the adsorption of peptides in the aqueous solution onto the plastic surface is suppressed by the sugar-based nonionic surfactant, a stable aqueous peptide solution preparation contained in a vessel having a plastic surface may be provided. Examples of the plastics include, but are not limited to, polypropylene, polystyrene, polymethyl methacrylate, polyvinyl chloride, polyethylene, polycarbonate, polysulfone, fluoropolymer, polyamide, polydimethylsiloxane, polyurethane, polysulfone, polytetrafluoroethylene, and elastomers. The plastic surface inside the container may or may not be subjected to hydrophilic treatment.

The aqueous solution of the present invention is useful as a sample for LC-MS analysis, a peptide reference standard, a peptide formulation for clinical or non-clinical use, or the like.

The definitions of the respective terms relating to the aqueous solution of the present invention are the same as those described for the production method of the present invention and the agent for accelerating dissolution of the present invention, unless otherwise specified.

### 4. Method for Preparing a Peptide-Containing Sample

In a further aspect, the present invention provides a method for preparing a peptide-containing sample (hereinafter referred to as "the sample preparation method of the present invention") comprising the following steps:
1) providing an aqueous peptide solution containing a peptide and 2,2-dodecylpropane-1,3-bis-β-D-maltopyranoside (lauryl maltose neopentyl glycol) (LMNG);
2) applying the obtained aqueous peptide solution to a reversed-phase column to allow the peptide and LMNG to be adsorbed onto the reversed-phase column; and
3) eluting the adsorbed peptide from the reversed-phase column while retaining LMNG adsorbed onto the reversed-phase column to remove LMNG from the peptide.

In the process of preparing a peptide-containing sample, when providing an aqueous peptide solution, adsorption of the peptide onto the vessel is suppressed by the inclusion of LMNG in the solution, thereby preventing the peptide loss. Moreover, LMNG strongly adsorbs to the reversed-phase column and has an elution condition significantly different from those of peptides. Therefore, even when peptides are eluted from the reversed-phase column under elution conditions capable of eluting most peptides including hydrophobic peptides, LMNG is retained in the column. Accordingly, LMNG can be readily separated from the peptides while avoiding peptide loss during the desalting step using the reversed-phase column. Since LMNG effectively suppresses peptide loss due to adsorption onto a vessel and can be readily removed from the peptides using a reversed-phase column, it is useful for the preparation of trace peptide-containing samples, such as peptide-containing samples for LC-MS analysis.

In the sample preparation method of the present invention, an aqueous peptide solution containing LMNG is first provided. The peptide may be a single species of peptide or a mixture of multiple species of peptides. In one embodiment, the peptide is a mixture of multiple species of peptides, and includes, at least in part, a long-chain peptide (for example, a peptide having a length of 11 amino acids or more, 12 amino acids or more, 13 amino acids or more, 14 amino acids or more, 15 amino acids or more, or 20 amino acids or more). The upper limit of the length of the long-chain peptide may be, for example, 100 amino acids or less, 80 amino acids or less, 60 amino acids or less, 50 amino acids or less, 40 amino acids or less, or 30 amino acids or less. The proportion (proportion of molecular number) of long-chain peptides in the peptide mixture is not particularly limited, and may be, for example, 0.1% or more, 1% or more, 5% or more, 10% or more, 20% or more, 30% or more, 40% or more, or 50% or more.

The origin of the peptide is not particularly limited, and it may be a peptide isolated from a biological material, chemically synthesized, or obtained as a recombinant product expressed by genetic engineering techniques. In one embodiment, the peptide to be dissolved in the aqueous solvent in the preparation method of the present invention is a peptide mixture obtained by limited digestion of a biological sample containing a protein using a protease (for example, trypsin). Although such a peptide mixture contains various peptides which differ in length and hydrophobicity, in the preparation method of the present invention, peptide adsorption onto the vessel is suppressed by the addition of LMNG, and during reversed-phase column purification, most peptides including long-chain peptides and hydrophobic peptides can be eluted while retaining LMNG adsorbed onto the reversed-phase column. Accordingly, a peptide mixture can be purified with high yield while maintaining the diversity of peptides in the sample.

The aqueous peptide solution in step (1) may be provided in a state contained in a vessel having a plastic surface. Adsorption of the peptide in the aqueous solution onto the plastic surface can be effectively suppressed by LMNG. Examples of plastics include those described in Section 1 above. The plastic surface inside the vessel may or may not be subjected to hydrophilic treatment. As used herein, "hydrophilic treatment" refers to a treatment that adds a hydrophilic group, such as a hydroxyl group or a carboxyl group, to the plastic surface inside the vessel.

The aqueous peptide solution may contain only LMNG as a surfactant, or may contain LMNG in combination with one or more other surfactants (for example, any of the sugar-based nonionic surfactants described above). In order to effectively separate the surfactant from the peptides by reversed-phase column treatment described below, it is preferable that the aqueous peptide solution contains only LMNG as the surfactant.

Additional components other than the peptides, LMNG, and water may be contained in the aqueous peptide solution. For example, buffering agents, salts (inorganic or organic salts), amino acids, and the like may be contained in the aqueous peptide solution. When preparing a peptide-containing sample for analysis by LC-MS or the like, it is preferable to avoid containing components that may interfere with the analysis or contaminate the analytical instrument.

The concentration of LMNG in the aqueous peptide solution may be a concentration that inhibits peptide adsorption to the vessel, for example, 0.00125 % (w/w) or more, 0.0025 % (w/w) or more, 0.005 % (w/w) or more, or 0.01 % or more.

Although it is not possible to unambiguously define an upper limit for the total concentration of LMNG contained in the aqueous peptide solution, the suppression of peptide adsorption onto a vessel does not require micelle formation by LMNG. Therefore, even when the concentration of LMNG is below its critical micelle concentration (CMC), the peptide adsorption can be suppressed. In one embodiment, the concentration of LMNG contained in the aqueous peptide solution is below the CMC of LMNG. Since LMNG can be removed from the peptide by reversed-phase column treatment as described later, contamination of the analytical instrument can be avoided when producing peptide-containing samples for analysis by LC-MS and the like. In one embodiment, the concentration of LMNG contained in the aqueous peptide solution is 0.04% (w/w) or less.

In one embodiment, the preparation method of the present invention may include obtaining an aqueous peptide solution containing a peptide and LMNG by subjecting a protein dissolved in an aqueous solvent containing LMNG to limited digestion with a protease.

The species of protein is not particularly limited, and protein-containing samples such as cell or tissue lysates, serum, or exosomes may be appropriately used. The protein may be an affinity-purified product such as an immunoprecipitate. For analysis by LC-MS, the protein may also be one in which cysteine residues have been subjected to reductive alkylation treatment using a reducing agent such as DTT or TCEP and an alkylating agent such as iodoacetamide. The protein may be modified such as phosphorylated, biotinylated, methylated, acetylated, glycosylated, or ubiquitinated.

The protease is not particularly limited, and examples thereof include trypsin, Glu-C, Lys-N, Lys-C, Asp-N, and chymotrypsin.

The concentration of LMNG in the aqueous protein solution to be subjected to enzymatic digestion (i.e., the concentration of LMNG in the aqueous solvent containing LMNG for dissolving the protein) may be a concentration that suppresses adsorption of the protein onto the vessel, and is, for example, 0.00125% (w/w) or more, 0.0025% (w/w) or more, 0.005% (w/w) or more, or 0.01% (w/w) or more. The upper limit of the concentration of LMNG contained in the aqueous protein solution cannot be unambiguously defined; however, suppression of protein adsorption onto the vessel does not require micelle formation by LMNG. In one embodiment, the concentration of LMNG contained in the aqueous protein solution is below the CMC of LMNG. In one embodiment, the concentration of LMNG contained in the aqueous protein solution is 0.04% (w/w) or less.

The aqueous protein solution may contain additional components other than the protein, LMNG, protease, and water. For example, a buffering agent suitable for protease digestion, salts (inorganic or organic salts), chaotropic agents, chelating agents, and the like may be contained in the aqueous protein solution. Examples of buffering agents suitable for trypsin digestion include Tris-HCl, ammonium bicarbonate, triethylammonium bicarbonate (TEAB) and the like. Examples of salt include sodium chloride (NaCl) and the like. Examples of chaotropic agents include urea, thiourea and the like. When preparing a peptide-containing sample for analysis by LC-MS or the like, it is preferable to avoid including components that may interfere with the analysis or contaminate the analytical instrument.

Before subjecting the aqueous peptide solution to reversed-phase column treatment, specific peptides may be enriched by affinity chromatography to obtain an aqueous peptide solution containing the affinity-enriched peptide and LMNG. Examples of affinity chromatography include, but are not limited to, immobilized metal affinity chromatography, avidin or streptavidin affinity chromatography and the like. The immobilized metal affinity chromatography includes both immobilized metal ion affinity chromatography and metal oxide affinity chromatography. For example, when phosphopeptides are contained in the peptides, the phosphopeptides may be enriched by applying the aqueous peptide solution to an immobilized metal ion affinity chromatography (IMAC) carrying metal ions such as Fe³⁺, or a metal oxide affinity chromatography carrying metal oxides such as TiO₂. Additionally, when biotinylated peptides are contained in the peptides, the biotinylated peptides may be enriched by applying the aqueous peptide solution to an avidin or streptavidin affinity chromatography. The inclusion of LMNG in the aqueous peptide solution may suppress non-specific binding of a peptide to the affinity column, thereby increasing the recovery of affinity-enriched peptides (e.g., phosphopeptides or biotinylated peptides). When performing such affinity purification, it is preferable to use an eluent added with LMNG and to collect the eluate as an aqueous peptide solution containing affinity-enriched peptides (e.g., enriched phosphopeptides or biotinylated peptides) and LMNG.

Subsequently, the resulting aqueous peptide solution is applied to a reversed-phase column. As a result, both peptides and LMNG are adsorbed onto the reversed-phase column. Additionally, by the reversed-phase column treatment, the aqueous peptide solution is desalted and salts, buffering agents, chaotropic agents and the like contained in the aqueous peptide solution are removed. Examples of reversed-phase columns include, but are not limited to, a column in which a hydrophobic group such as octadecyl group (C18), octyl group (C8), butyl group (C3), phenyl group, or cyanopropyl group is bonded to solid carriers (e.g., silica gel), and styrene-divinylbenzene (SDB) copolymer column. The reversed-phase column is preferably a column in which octadecyl group (C18) is bonded to solid carriers (e.g., silica gel) (commonly referred to as a C18 column) or an SDB copolymer column. Commercially available reversed-phase columns may be used, and commercially available kits such as EVOSEP ONE and SDB-STAGE tips may also be employed.

Subsequently, the peptides adsorbed onto the reversed-phase column are eluted. Since LMNG has a higher affinity for the reversed-phase column than the peptides, the peptides adsorbed onto the reversed-phase column can be eluted while retaining LMNG adsorbed onto the column, thereby allowing LMNG to be removed from the peptides. The solvent used for peptide elution is typically a mixed solvent of an organic solvent such as acetonitrile, methanol, tetrahydrofuran, isopropanol, or acetone, and water. To promote protonation, it is preferable to add an acid such as trifluoroacetic acid, formic acid, acetic acid, hydrochloric acid or the like to the water. The acid concentration is typically 0.05% to 0.2% (v/v). To minimize the contamination of LMNG into the eluted peptides, the polarity of the eluent for peptide elution is preferably equivalent to or greater than that of a mixed solution of acetonitrile / 0.1% (v/v) trifluoroacetic acid aqueous solution = 50/50 (v/v); for example, equivalent to or greater than a mixed solution of acetonitrile / 0.1% (v/v) trifluoroacetic acid aqueous solution = 40/60 (v/v), equivalent to or greater than that of a mixed solution of acetonitrile / 0.1% (v/v) trifluoroacetic acid aqueous solution = 38/62 (v/v), or equivalent to or greater than that of a mixed solution of acetonitrile / 0.1% (v/v) trifluoroacetic acid aqueous solution = 37/63 (v/v). On the other hand, from the viewpoint of minimizing peptide retention on the reversed-phase column and increasing peptide recovery, the polarity of the eluent for peptide elution is preferably lower; typically, it is equivalent to or less than that of a mixed solution of acetonitrile / 0.1% (v/v) trifluoroacetic acid aqueous solution = 30/70 (v/v), preferably equivalent to or less than a mixed solution of acetonitrile / 0.1% (v/v) trifluoroacetic acid aqueous solution = 32/68 (v/v), equivalent to or less than a mixed solution of acetonitrile / 0.1% (v/v) trifluoroacetic acid aqueous solution = 34/66 (v/v), equivalent to or less than a mixed solution of acetonitrile / 0.1% (v/v) trifluoroacetic acid aqueous solution = 35/65 (v/v), or equivalent to or less than a mixed solution of acetonitrile / 0.1% (v/v) trifluoroacetic acid aqueous solution = 36/64 (v/v). In one embodiment, the polarity of the eluent is set to be equivalent to or less than that of a mixed solution of acetonitrile / 0.1% (v/v) trifluoroacetic acid aqueous solution = 30/70 (v/v) and equivalent to or greater than that of a mixed solution of acetonitrile / 0.1% (v/v) trifluoroacetic acid aqueous solution = 50/50 (v/v). In one embodiment, the polarity of the eluent is set to be equivalent to or less than that of a mixed solution of acetonitrile / 0.1% (v/v) trifluoroacetic acid aqueous solution = 30/70 (v/v) and equivalent to or greater than that of a mixed solution of acetonitrile / 0.1% (v/v) trifluoroacetic acid aqueous solution = 40/60 (v/v). In one embodiment, the polarity of the eluent is set to be equivalent to or less than that of a mixed solution of acetonitrile / 0.1% (v/v) trifluoroacetic acid aqueous solution = 30/70 (v/v) and equivalent to or greater than that of a mixed solution of acetonitrile / 0.1% (v/v) trifluoroacetic acid aqueous solution = 38/62 (v/v). A typical eluent used for elution from a C18 column used in EVOSEP ONE is a mixed solution of acetonitrile / 0.1% (v/v) acetic acid aqueous solution = 35/65 (v/v). The polarity of the eluent can be calculated based on the Snyder polarity parameters unique to each solvent and their mixing ratios. As used herein, "a mixed solution of acetonitrile / 0.1% (v/v) trifluoroacetic acid aqueous solution = 30/70 (v/v)" means a mixed solution obtained by mixing 30 parts by volume of acetonitrile and 70 parts by volume of 0.1% (v/v) trifluoroacetic acid aqueous solution, and other mixtures are interpreted in the same manner.

Through this reversed-phase column treatment, LMNG can be removed from the peptides, thereby providing a purified peptide-containing sample. This peptide-containing sample is useful for LC-MS analysis, and the eluted peptides can be dried (for example, dried in a vessel having a plastic surface) and redissolved in water to be subjected to LC-MS analysis.

Accordingly, in one embodiment, the preparation method of the present invention can also be regarded as a method for identifying a peptide, comprising the following steps :
1) providing an aqueous peptide solution containing a peptide and 2,2-didecylpropane-1,3-bis-β-D-maltopyranoside (lauryl maltose neopentyl glycol) (LMNG);
2) applying the obtained aqueous peptide solution to a reversed-phase column to allow the peptide and LMNG to be adsorbed onto the reversed-phase column;
3) eluting the adsorbed peptide from the reversed-phase column while retaining LMNG adsorbed onto the reversed-phase column to remove LMNG from the peptide;
4) drying the eluted peptide to obtain a dried peptide;
5) dissolving the obtained dried peptide in water to obtain an aqueous peptide solution; and
6) subjecting the resulting aqueous peptide solution to LC-MS analysis to identify the peptide based on the MS analysis results.

In another embodiment, the preparation method of the present invention can also be regarded as a method for identifying a protein in a protein-containing sample (a method for analyzing a protein profile or a method for conducting a proteome analysis), comprising the following steps:
1) dissolving a protein in a protein-containing sample in an aqueous solvent containing LMNG and subjecting the protein to limited digestion with a protease to obtain an aqueous peptide solution containing peptides and LMNG;
2) applying the obtained aqueous peptide solution to a reversed-phase column to allow the peptides and LMNG to be adsorbed onto the reversed-phase column;
3) eluting the adsorbed peptides from the reversed-phase column while retaining LMNG adsorbed onto the reversed-phase column to remove LMNG from the peptides;
4) drying the eluted peptides to obtain dried peptides;
5) dissolving the obtained dried peptides in water to obtain an aqueous peptide solution;
6) subjecting the obtained aqueous peptide solution to LC-MS analysis to identiry the peptides based on the MS analysis results; and
7) identifying the protein in the protein-containing sample based on the identified peptides.

In addition, by dissolving the dried peptide by the above-described production method of the present invention, the dissolution of the dried peptide is accelerated, and the loss of peptide during the redissolution step can be suppressed, thereby further increasing the number of identified peptides and proteins in LC-MS. That is, the present invention also provides a method for preparing a peptide-containing sample, comprising the following steps:
1. providing an aqueous peptide solution containing a peptide and LMNG;
2. applying the obtained aqueous peptide solution to a reversed-phase column to allow the peptide and LMNG to be adsorbed onto the reversed-phase column;
3. eluting the adsorbed peptide from the reversed-phase column while retaining LMNG adsorbed onto the reversed-phase column to remove LMNG from the peptide;
4. drying the eluted peptide to obtain a dried peptide; and
5. dissolving the obtained dried peptide in an aqueous solvent containing at least one sugar-based nonionic surfactant to obtain an aqueous peptide solution.

In one embodiment, the present invention also provides a method for identifying a peptide, comprising the following steps:
1) providing an aqueous peptide solution containing a peptide and 2,2-didecylpropane-1,3-bis-β-D-maltopyranoside (lauryl maltose neopentyl glycol) (LMNG);
2) applying the obtained aqueous peptide solution to a reversed-phase column to allow the peptide and LMNG to be adsorbed onto the reversed-phase column;
3) eluting the adsorbed peptide from the reversed-phase column while retaining LMNG adsorbed onto the reversed-phase column to remove LMNG from the peptide;
4) drying the eluted peptide to obtain a dried peptide;
5) dissolving the resulting dried peptide in an aqueous solvent containing at least one sugar-based nonionic surfactant to obtain an aqueous peptide solution; and
6) subjecting the resulting aqueous peptide solution to LC-MS analysis and identifying the peptide based on the MS analysis results.

In another embodiment, the present invention also provides a method for identifying a protein in a protein-containing sample (a method for analyzing a protein profile or a method for conducting proteome analysis), comprising the following steps:
1) dissolving a protein in a protein-containing sample in an aqueous solvent containing LMNG and subjecting the protein to limited digestion with a protease to obtain an aqueous peptide solution containing peptides and LMNG;
2) applying the obtained aqueous peptide solution to a reversed-phase column to allow the peptides and LMNG to be adsorbed onto the reversed-phase column;
3) eluting the adsorbed peptides from the reversed-phase column while retaining LMNG adsorbed onto the reversed-phase column to remove LMNG from the peptides;
4) drying the eluted peptides to obtain dried peptides;
5) dissolving the obtained dried peptides in an aqueous solvent containing at least one sugar-based nonionic surfactant to obtain an aqueous peptide solution;
6) subjecting the obtained aqueous peptide solution to LC-MS analysis to identify the peptides based on the MS analysis results; and
7) identifying the protein in the protein-containing sample based on the identified peptides.

The definitions of the respective terms regarding the sample preparation method of the present invention are the same as those described for the production method of the present invention, the agent for accelerating dissolution of the present invention, and the aqueous peptide solution of the present invention, unless otherwise specified.

All references cited in the present specification, including publication, patent document and the like, are hereby incorporated individually and specifically by reference, to the extent that the entireties thereof have been specifically disclosed herein.

The present invention is explained in more detail in the following by referring to Examples, which are not to be construed as limitative.

### EXAMPLES

### [Example 1] Dissolution of dried peptide mixture with DDM solution

### (Materials and methods)

### Dried peptide mixture

100 µg of K562 cell tryptic digest (CAT# V6951, Promega, Madison, WI, USA) was adjusted to 50 ng/µL with 50% acetonitrile (CAN) containing 0.1% trifluoroacetic acid (TFA), and dispensed in 10 µL each into normal tubes (1.5-ml safe-lock tube, CAT# 0030120086, Eppendorf, Hamburg; Germany), normal polypropylene (PP) vials (CAT# C5000-97, Thermo Fisher Scientific, Waltham, MA, USA) or hydrophilic vials (ProteoSave vial, CAT# 11-19-1021-10, AMR Inc., Tokyo, Japan). These samples were dried in a centrifugal evaporator (miVac Duo concentrator, Genevac Ltd., Ipswich, UK), and redissolved in 25 µL of 2% ACN containing 0.1% TFA or 0.005-0.04% DDM.

### Co-immunoprecipitation (coIP) and on-beads digestion

HeLa cells were mixed with IP lysis buffer (Pierce IP Lysis Buffer (CAT# 87788, Thermo Fisher Scientific, Waltham, MA, USA) containing protease inhibitors (CAT# 5892791001, cOmplete ULTRA Tablets, Sigma-Aldrich, MO, USA) and phosphatase inhibitors (CAT# 4906837001, PhosSTOP Tablets, Sigma-Aldrich) for 30 sec, then incubated in ice for 30 min and mixed for 30 sec. The cell lysate was then centrifuged at 18,000g at 4 °C for 30 min and the supernatant was collected. Protein concentration in the protein extract was determined using a BCA protein assay kit (CAT# 23225, Thermo Fisher Scientific) and adjusted to 2 µg/µL with the IP lysis buffer.

Co-Immunoprecipitation (IP) from the HeLa cell lysate was carried out with automated TANBead Maelstrom 8 (TANBead, Taipei, Taiwan). SpeedBeads Protein A/G Magnetic Particles (CAT# 17152104010150, Cytiva, Uppsala, Sweden) were used as beads for Co-IP, and anti-RELA (NF-κB p65) antibody (CAT# ab16502, Abcam, Cambridge, MA, USA) was used as the antibody. To prepare the beads for immunoprecipitation, 1.5 µL of bead slurry was washed once with 500 µL of tris-buffered saline containing 0.05% Tween20 (TBST). Then the anti-RELA antibody was captured on the beads by mixing the beads at room temperature for 30 min in 200 µL of TBST containing 4 µg anti-RELA antibody and washing twice in 500 µL of TBST to remove unbound antibody. Subsequently, 200 µL of HeLa cell lysate (2 µg/uL) was added to the beads and incubated at room temperature for 60 min with end-over mixing. The beads were washed twice with 500 µL of IP lysis buffer, once with 500 µL of TBST and once with 500 µL of 50 mM Tris-HCl (pH 8.0). Subsequently, 100 µL of 50 mM Tris-HCl (pH 8.0) was added to the beads, and on-bead digestion was performed with a slight modification to a previously reported procedure (Nat Cell Biol 2018, 20 (1), 81-91). Briefly, 500 ng of trypsin/Lys-C Mix (CAT# V5072, Promega, Madison, WI, USA) was added to the beads, and protein digestion was carried out overnight at 37°C with gentle mixing. The beads were then aggregated from the digested sample using a magnetic stand (EpiMag HT (96-Well) Magnetic Separator, EpiGentek, Brooklyn, NY, USA) to collect the supernatant. The collected sample was treated with 20 mM tris(2-carboxyethyl)phosphine at 80 °C for 10 min, followed by alkylation with 35 mM iodoacetamide at room temperature for 30 min under light-protected conditions. Subsequently, the alkylated sample was acidified with 20 µL of 5% trifluoroacetic acid (TFA), desalted using a STAGE tip (CAT# 7820-11200, GL Sciences Inc., Tokyo, Japan) according to the manufacturer's protocol, and dried in a centrifugal evaporator (miVac Duo concentrator, Genevac Ltd., Ipswich, UK). The dried sample was redissolved in 12 µL of either 2% acetonitrile (ACN) with 0.1% TFA or 0.02% DDM and transferred to normal PP vials (Thermo Fisher Scientific). An aliquot of 2.5 µL of the sample was injected into the LC-MS/MS.

### LC-MS/MS

The redissolved peptides were directly injected onto a 75 µm × 12 cm nanoLC nano-capillary column (Nikkyo Technos Co., Ltd., Tokyo, Japan) at 40 °C and then separated with a 60 min gradient (A = 0.1% FA in water, B = 0.1% FA in 80% ACN) consisting of 0 min 8% B, 60 min 44% B (for coIP-MS) and 0 min 6% B, 60 min 48% B (for other than coIP-MS) at a flow rate of 150 nl/min using an UltiMate 3000 RSLCnano LC system (Thermo Fisher Scientific, Waltham, MA, USA). The peptides eluted from the column were analyzed on an Orbitrap Exploris 480 (Thermo Fisher Scientific) with DDA and overlapping window DIA modes (J Am Soc Mass Spectrom 2019, 30 (4), 669-684). In DDA mode, MS1 spectra were acquired over the range of 380-1,240 m/z with a resolution of 60,000, an auto gain control (AGC) target of 3 × 10⁶, and a maximum injection time of 100 msec. The 40 most intense ions with charge states of 2+ to 6+ that exceeded 8.0 × 10³ were fragmented by collision-induced dissociation with a normalized collision energy of 30%. MS2 spectra were acquired above 200 m/z with a resolution of 15,000, an AGC target of 1 × 10⁵, and a maximum injection time of 50 msec. The dynamic exclusion time was set to 20 sec. In overlapping window DIA mode, MS1 spectra were acquired over the range of 495-905 m/z with a resolution of 30,000, an AGC target of 3 × 10⁶, and a maximum injection time of 55 msec. MS2 spectra were acquired over the range of 200-1,800 m/z with a resolution of 30,000, an AGC target of 3 × 10⁶, and a maximum injection time set to "auto". Stepped normalized collision energy was set to 28%. The isolation width for MS2 was set to 8 m/z, and for the 500-900 m/z overlap window pattern, an optimized window arrangement was used in Xcalibur 4.4 (Thermo Fisher Scientific).

The DDA-MS files were searched against the human UniProt reference proteome (Proteome ID UP000005640, reviewed, canonical; 20,381 entries). For DDA-MS files, the Proteome Discoverer 2.5 (Thermo Fisher Scientific) search engine was used with Sequest HT and Percolator. The setting parameters were as follows:

| | |
|---|---|
| Enzyme | Trypsin |
| Maximum missed cleavage sites | 3 |
| Precursor mass tolerance | 10 ppm |
| Fragment mass tolerance | 0.02 Da |
| Static modification | Cysteine carbamidomethylation |
| Dynamic modification | Methionine oxidation |

The DIA-MS files were searched against an in silico human spectral library using Scaffold DIA (Proteome Software, Inc., Portland, OR, USA), as previously reported (J Proteome Res 2022, 21(5), 1340-1348).

### Data analysis

Pearson correlation coefficient heatmap analysis with principal component analysis (PCA) and hierarchical clustering was performed using Perseus v1.6.15.0 (Nat Methods 2016, 13 (9), 731-740).

### (Results and discussion)

### Dissolution of dried peptides by DDM

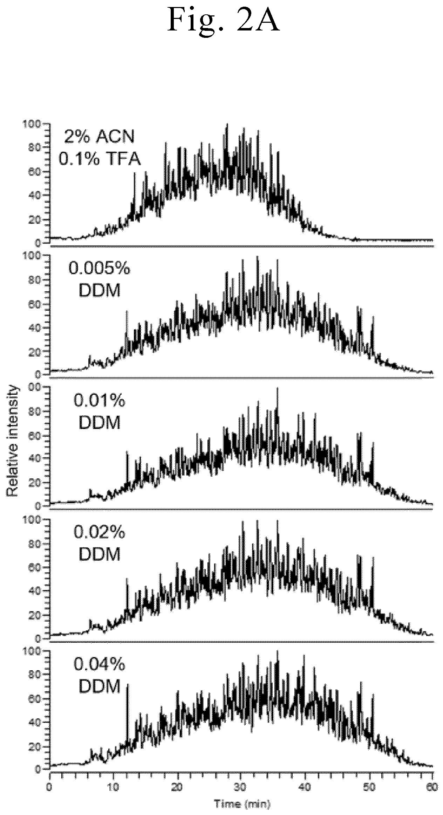

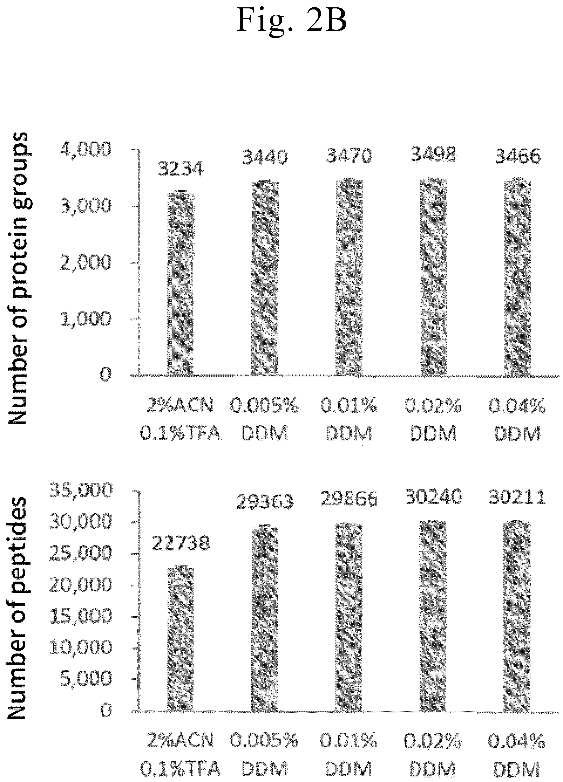

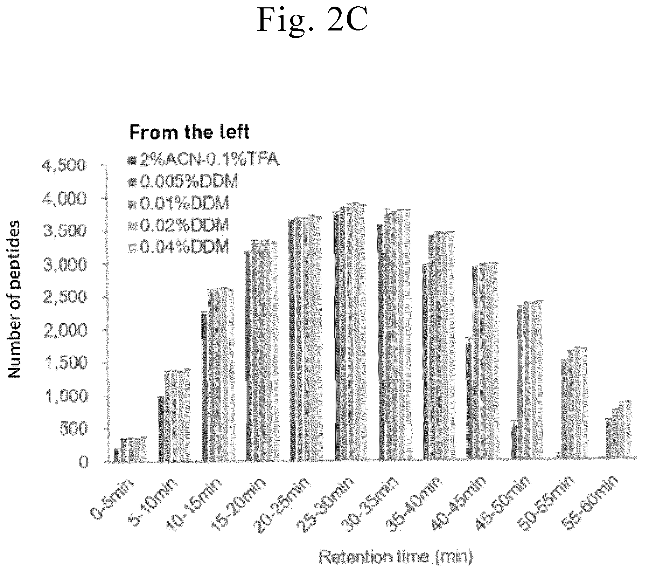

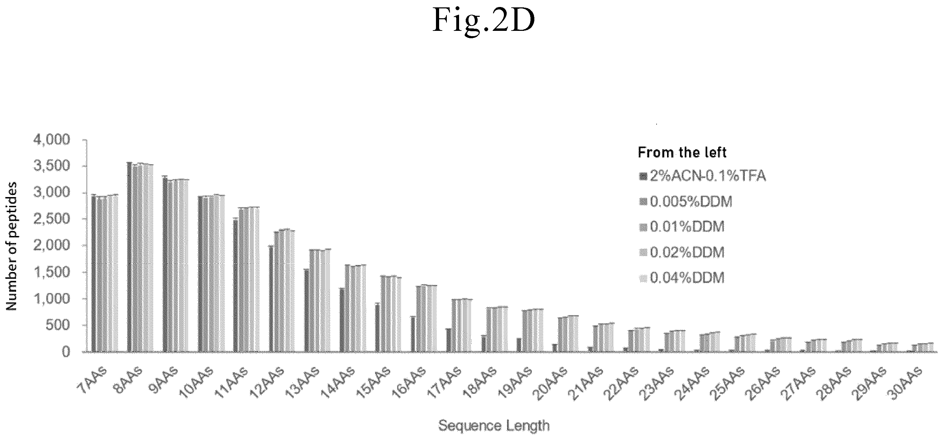

To investigate the effect of DDM solution on the recovery rate of dried peptides, a small amount (500 ng) of dried tryptic peptides, which are prone to loss, was prepared. The dried peptides were dissolved in a conventional 2% CAN-0.1% TFA without any surfactant, or in 0.005-0.04% DDM, and subjected to DDA-LC-MS/MS analysis. Compared to the 0.005-0.04% DDM, the 2% CAN-0.1% TFA exhibited clearly lower peak intensities after a retention time of 40 min (Figure 2A), and resulted in fewer identified protein groups and peptides (Figure 2B). The primary reason for the smaller number of protein groups and peptides identified in the 2% CAN-0.1% TFA is the marked decrease in the number of peptides identified after the 40 min retention time (Figure 2C). When analyzing the length of peptides identified from each solution, it was found that the difference in the number of peptide identifications between the 2% CAN-0.1% TFA and DDM solutions increased with increasing peptide length (Figure 2D). These results demonstrated that the use of DDM primarily improves recovery of long peptides. Although slight differences were observed depending on the DDM concentration, 0.02% identified the greatest number of protein groups and peptides; therefore, 0.02% DDM solution was used for peptide dissolution in subsequent experiments.

### Change in recovery of dried peptides by hydrophilic vials

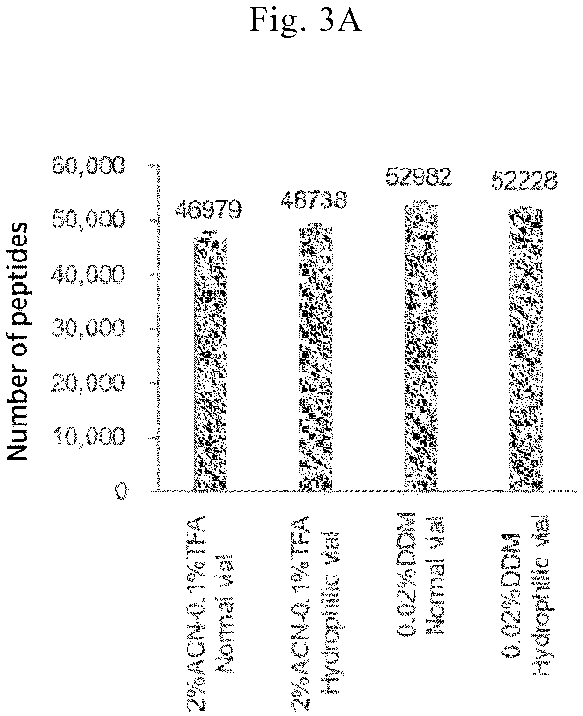

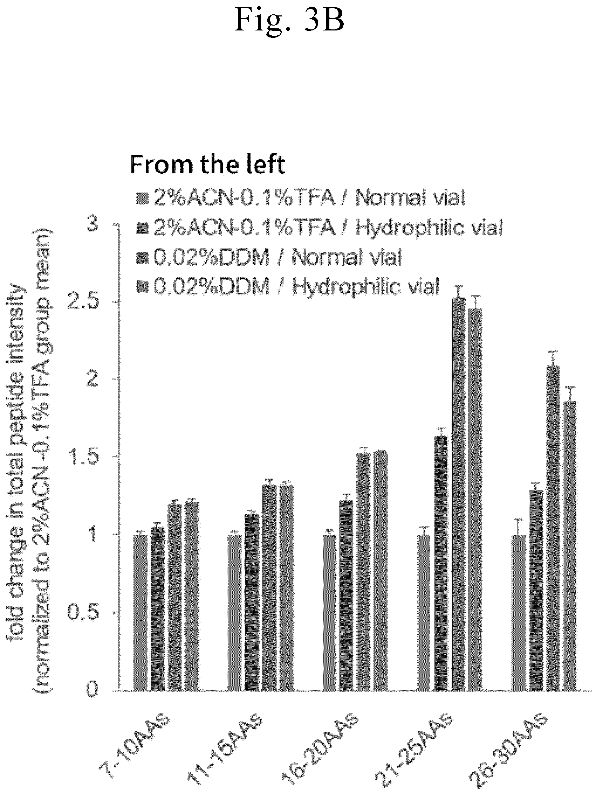

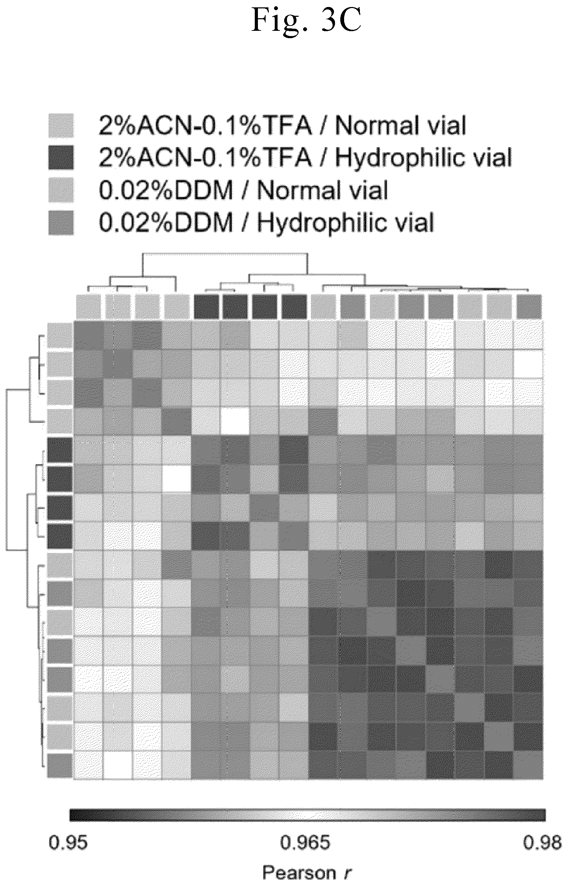

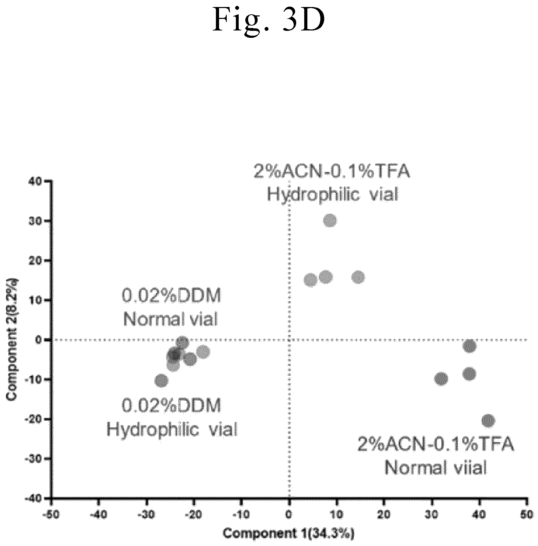

The recovery of dried peptides was evaluated in vials with a hydrophilic coating. 500 ng of tryptic peptides were dried either in a hydrophilic-coated vial or a normal polypropylene (PP) vial, dissolved in either 2% ACN-0.1% TFA or 0.02% DDM, and subjected to DIA-LC-MS/MS analysis for quantitative evaluation. When dissolved in 2% CAN-0.1% TFA, a greater number of peptides were identified in the hydrophilic-coated vials, whereas a slightly lower number was identified in the hydrophilic-coated vial when dissolved in 0.02% DDM (Figure 3A). Regardless of the vial type, peptide identification was consistently higher when using 0.02% DDM compared to 2% ACN-0.1% TFA. Regarding peptide intensity, the highest peak intensities were observed in the following order: 0.02% DDM in a normal vial, 0.02% DDM in a hydrophilic-coated vial, 2% CAN-0.1% TFA in a hydrophilic-coated vial, and 2% CAN-0.1% TFA in a normal vial, with the difference becoming more pronounced for longer peptides (Figure 3B). Cluster analysis based on protein intensity indicated that, with DDM, clustering patterns were not separated significantly between vial types, whereas separated between vials with 2% CAN-0.1% TFA. Notably, the cluster derived from hydrophilic-coated vials using 2% CAN-0.1% TFA was more similar to the case of dissolution with DDM (Figure 3C). A similar trend was observed in principal component analysis based on protein intensity (Figure 3D). Both peptide-level and protein-level analyses demonstrated that the use of hydrophilic-coated vials for dissolution with 2% CAN-0.1% TFA improved the recovery of dried peptides. On the other hand, in dissolution with 0.02% DDM, the use of hydrophilic-coated vial resulted in a slight decrease in peptide recovery in the peptide-level analysis, whereas no significant difference was observed between the two types of vials in the protein-level analysis. The presence or absence of the use of 0.02% DDM had a more significant impact on peptide recovery than the difference in the vial type, suggesting that the normal vial is sufficient when using 0.02% DDM. Hydrophilic-coated vials and tubes are expensive; however, since high peptide recovery can be maintained even with normal PP vials or tubes by dissolving the peptides with inexpensive DDM, this approach is advantageous in terms of cost. Although DDM is non-volatile and may pose a risk of contaminating MS instruments, the frequency of instrument cleaning has not changed over the six-month period during which DDM has been used, as compared to when 2% ACN-0.1% TFA was used. It is considered that the use of DDM at a low concentration of 0.02% may have reduced contamination of the MS instrument.

### Effect of DDM on coIP-MS

Proteins that are bound to low-abundance proteins, such as transcription factors, are present in even smaller amounts, which has made it difficult to search for interactors of such proteins by coIP-MS. In this study, to observe such trace interactors by coIP-MS, peptides were attempted to be dissolved in DDM after desalted with a reversed-phase spin column and dried with a centrifugal evaporator. In the coIP experiment, an antibody against RELA (NF-κB p65), a transcription factor, was used to react with HeLa lysate. Figure 4A shows the TIC chromatograms obtained by dissolving the dried tryptic peptides obtained by coIP against RELA in either 2% CAN-0.1% TFA or 0.02% DDM and analyzing them by DIA-LC-MS/MS (Figure 4A). As with the results shown in Figure 2A, the peptides dissolved in 0.02% DDM exhibited clearly higher peak intensities during the later retention time range of the LC compared to those dissolved in 2% ACN-0.1% TFA. The number of identified protein groups and peptides was also higher when 0.02% DDM was used (Figure 4B). Furthermore, the number of known RELA-interactors in the identified protein groups was also higher in the 0.02% DDM sample (Figure 4C). These results indicate that merely changing the solvent used to dissolve dried peptides to 0.02% DDM allowed for the detection of a greater number of interactors. In addition, investigation of the protein intensities of RELA and its well-known interactors (NFKB1, NFKB2, NFKBIA, NFKBIB, and CREBBP) revealed that all proteins showed higher intensities in the 0.02% DDM-dissolved sample, indicating improved recovery of these important proteins in coIP-MS for RELA (Figure 4D). Thus, dissolving dried peptides in DDM in coIP-MS was highly effective for improving peptide recovery and enabling the detection of many interaction partners. DDM can improve the recovery of dried peptides in other proteomic approaches, especially when dealing with trace samples.

### (Conclusion)

The effect of DDM solution was investigated to improve the recovery of dried peptides. The use of DDM significantly improved the recovery of long-chain peptides. Furthermore, by using DDM, a high recovery of peptides was achieved even in a normal PP vial, without the need for expensive hydrophilic-coated vial. This method was applied to coIP-MS analysis of RELA, demonstrating improved peptide recovery and the detection of many interactors. Since the recovery, particularly of long-chain peptides, was improved simply by switching the solvent for dissolving peptides to DDM, the method is considered readily applicable to a wide range of proteomics approaches.

### [Example 2] Dissolution of dried peptide mixtures with various sugar-based nonionic surfactant solutions

In the same manner as in Example 1, 400 ng of the dried peptide mixture was dissolved in 20 µL of various sugar-based nonionic surfactant solutions at concentrations ranging from 0.00125 to 0.04%, and 2.5 µL of the resulting peptide solution was analyzed by LC-MS/MS. The evaluated sugar-based nonionic surfactants are listed in Table 2.

**[Table 2]**

| **ID** | **Type of Sugar-Based Surfactants** | **Name of Surfactants** | **Cas no** | **MW** |
|---|---|---|---|---|
| A | Maltose | n-Dodecyl- *β* -D-maltoside | 69227-93-6 | 510.62 |
| B | Maltose | n-Undecyl- *β* -D-maltoside | 253678-67-0 | 496.59 |
| C | Maltose | n-Decyl- *β* -D-maltoside | 82494-09-5 | 482.57 |
| D | Maltose | n-Nonyl- *β* -D-thiomaltoside | 148565-55-3 | 484.6 |
| E | Glucose | n-Octyl- *β* -D-thioglucoside | 85618-21-9 | 308.44 |
| F | Mannose | 3-Oxatridecyl- *α* -D-mannoside | 914802-92-9 | 364.47 |
| G | Neopentyl Glycol | Decyl Maltose Neopentyl Glycol | 1257852-99-5 | 949.08 |
| H | Neopentyl Glycol | Lauryl Maltose Neopentyl Glycol | 1257852-96-2 | 1005.19 |
| I | Neopentyl Glycol | Octyl Glucose Neopentyl Glycol | 1257853-32-9 | 568.69 |
| J | Trehalose Ester | Trehalose C12 | 64622-91-9 | 524.6 |

The number of peptides that could be identified by LC-MS/MS is shown in Table 3 and Figure 5.

**[Table 3]**

| **Conc.(%)** | **A** | **B** | **C** | **D** | **E** | **F** | **G** | **H** | **I** | **J** |
|---|---|---|---|---|---|---|---|---|---|---|
| **0.04** | 18826 | 18834 | 18567 | 18253 | 18128 | 18764 | 19188 | 18718 | 19178 | 19126 |
| **0.02** | 18752 | 18860 | 18191 | 17427 | 17481 | 19287 | 19078 | 18629 | 19254 | 19029 |
| **0.01** | 18500 | 18782 | 17125 | 16708 | 15647 | 18956 | 18901 | 18486 | 18772 | 18709 |
| **0.005** | 18270 | 17541 | 16385 | 16157 | 14907 | 18261 | 18536 | 18248 | 18258 | 18241 |
| **0.0025** | 16810 | 16658 | 15274 | 14349 | 14022 | 17764 | 17624 | 17324 | 16364 | 16602 |
| **0.00125** | 14566 | 13846 | 14207 | 13979 | 13871 | 15970 | 14752 | 15501 | 15845 | 14477 |

When the dried peptide mixture was dissolved in each 0.04% sugar-based nonionic surfactant solution, the number of identified peptides was improved compared to 2% ACN-0.1% TFA with any of the sugar-based nonionic surfactants (Figure 5), indicating that the sugar-based nonionic surfactant is effective in accelerating the dissolution of the dried peptide mixtures. Even when the surfactant concentration was reduced to 0.0025%,
A (n-dodecyl-β-D-maltoside),
B (n-undecyl-β-D-maltoside),
F (3-oxatridecyl-α-D-mannoside),
G (decyl maltose neopentyl glycol),
H (lauryl maltose neopentyl glycol),
I (octylglucose neopentyl glycol) and
J (trehalose C12)
maintained a high number of identified peptides (Table 3). Among these, surfactant F (3-oxatridecyl-α-D-mannoside) exhibited a particularly high number of identified peptides (Table 3).

### [Example 3] Acceleration of solubility of a dried peptide mixture by combination of Two Surfactants

In Example 2, six sugar-based nonionic surfactants (A, F, G, H, I, and J) with the highest number of identified peptides at a concentration of 0.005% were selected, and their effect on the solubility of a dried peptide mixture was evaluated when two of these surfactants were combined. Aqueous solutions of the two surfactant mixtures were prepared so that the final concentration of each surfactant was 0.005% and the total concentration of the two surfactants was 0.01%. In the same manner as in Example 2, 400 ng of a dried peptide mixture was dissolved in 20 µL of each surfactant solution, and 2.5 µL of the resulting peptide solution was subjected to LC-MS/MS analysis to compare the number of identified peptides. The results are shown in Table 4.

**[Table 4]**

| | | | | | |
|---|---|---|---|---|---|
| **G** | 19105 | 19622 | | | |
| **H** | 18801 | - | 19139 | | |
| **I** | 19144 | 19347 | 19515 | 19212 | |
| **J** | 19505 | 19265 | 19180 | - | 19403 |
| | **A** | **F** | **G** | **H** | **I** |

When two sugar-based nonionic surfactants were used in combination (A-G, A-H, A-I, A-J, F-G, F-I, F-J, G-H, G-I, G-J, H-I, I-J) at a total concentration of 0.01%, the number of identified peptides was greater than when each surfactant was used alone at a concentration of 0.01% (Table 3), as shown in Table 4, suggesting that the combination of two sugar-based nonionic surfactants synergistically accelerates the dissolution of dried peptides. In particular, the combinations of A-J, J-I, G-I, and F-G resulted in particularly high numbers of identified peptides. The number of identified peptides obtained using these four combinations at a concentration of 0.01% was compared with those obtained using the top six individual surfactants (A, B, F, G, I, J) that showed a higher number of identified peptides at a concentration of 0.01%. The results are shown in Table 5.

**[Table 5]**

| **Surfactants** | **A** | **B** | **F** | **G** | **I** | **J** | **A-J** | **J-I** | **G-I** | **F-G** |
|---|---|---|---|---|---|---|---|---|---|---|
| **Conc.(%)** | **0.01** | **0.01** | **0.01** | **0.01** | **0.01** | **0.01** | **0.005-0.005** | **0.005-0.005** | **0.005-0.005** | **0.005-0.005** |
| **Exp. 1** | 19612 | 19405 | 19498 | 19501 | 19470 | 19161 | 19945 | 19732 | 19734 | 19847 |
| **Exp. 2** | 19519 | 19325 | 19767 | 19389 | 19550 | 19242 | 20106 | 19632 | 19886 | 19866 |
| **Exp. 3** | 19435 | 19140 | 19878 | 19448 | 19355 | 19330 | 20129 | 19518 | 19864 | 19906 |
| **Mean** | 19522 | 19290 | 19714 | 19446 | 19458 | 19244 | 20060 | 19627 | 19828 | 19873 |
| **S.D.** | 72 | 111 | 160 | 46 | 80 | 69 | 82 | 87 | 67 | 25 |

As a result, the combination of A and J yielded the highest number of identified peptides, suggesting that this combination is the most suitable surfactant combination for dissolving dried peptides.

### [Example 4] Improvement of peptide loss by LMNG and elimination thereof

### (Materials and methods)

### LC-MS measurement of surfactants

The surfactants were directly injected onto a 75 µm × 12 cm nanoLC column (Nikkyo Technos Co., Ltd., Tokyo, Japan) at 50 °C and separated with a 30 min gradient (A = 0.1% formic acid in water, B = 0.1% formic acid in 80% acetonitrile), consisting of 5% B at 0 min, 95% B at 25 min, and 95% B at 30 min, at a flow rate of 300 nL/min using an UltiMate 3000 RSLCnano LC system (Thermo Fisher Scientific). The eluates from the column were analyzed by DDA on a Q Exactive HF-X (Thermo Fisher Scientific). MS1 spectra were collected over the range of 100-1,500 m/z with a resolution of 60,000, with the automatic gain control (AGC) target set to 1 × 10⁶ and the maximum injection time set to 119 ms. MS chromatograms of the monoisotopic masses (singly charged) of each surfactant were obtained.
FA: Formic acid
ACN: Acetonitrile

### Elution conditions of LMNG, DMNG and DDM from SDB-STAGE tip

The SDB-STAGE tip was washed with 25 µL of 80% acetonitrile (ACN) in 0.1% trifluoroacetic acid (TFA), followed by equilibration with 50 µL of 3% ACN in 0.1% TFA. Subsequently, 10 µL of 0.001% LMNG, DMNG or DDM was loaded onto the tip, the tip was washed with 80 µL of 3% ACN in 0.1% TFA, and the sample was then eluted stepwise with 50 µL each of 30% ACN in 0.1% TFA, 40% ACN in 0.1% TFA, and 50% ACN in 0.1% TFA. An additional DMNG sample was further eluted stepwise with 50 µL of 30% ACN in 0.1% TFA, 32% ACN in 0.1% TFA, 34% ACN in 0.1% TFA, 36% ACN in 0.1% TFA, 38% ACN in 0.1% TFA, and 40% ACN in 0.1% TFA. The eluates were dried using a centrifugal evaporator (miVac Duo concentrator, Genevac Ltd., Ipswich, UK). The dried samples were redissolved in 200 µL of H₂O and transferred to a normal vial (CAT# C5000-97, Thermo Fisher Scientific, Waltham, MA, USA).

### Protein extraction from HEK293T cells

Proteins were extracted from HEK293T cells by sonication for 10 min using a Bioruptor II (CosmoBio, Tokyo, Japan) in a solution containing 4% sodium dodecyl sulfate (SDS), 100 mM Tris-HCl (pH 8.0), and 20 mM NaCl. The protein concentration in the extract was determined using a BCA protein assay kit (CAT# 23225, Thermo Fisher Scientific), and adjusted to 500 ng/µL or 5 ng/µL using the same buffer containing 4% SDS, 100 mM Tris-HCl (pH 8.0), and 20 mM NaCl.

### Purification of extracellular vehicles (EVs) from human serum

Purification of human serum EVs was performed using a Tim4-phosphatidylserine (PS) affinity method in combination with the MagCapture Exosome Isolation Kit PS Ver.2 (Wako Pure Chemical, Osaka, Japan) and the Maelstrom 8 Autostage (TANBeads). Briefly, 250 µL of pooled serum from healthy human donors was centrifuged at 3,000 × g for 20 min at 4°C, and 200 µL of the supernatant was collected into a new tube. Then, 300 µL of TBS and 1 µL of Exosome Binding Enhancer (provided with the kit) were added to the supernatant and gently mixed. To prepare beads for the Tim4-PS affinity method, 30 µl of Exosome Capture Beads (provided with the kit) were washed once with 250 µl of Exosome Immobilizing/Washing Buffer (provided with the kit). The beads were then mixed with 10 µL of biotin-labeled Exosome Capture reagent (diluted with 250 µL of Exosome Immobilizing/Washing Buffer) and agitated at 1,000 rpm for 10 min. After washing twice with 250 µl of Exosome Immobilizing/Washing Buffer, the beads were added to the sample. To allow EVs to bind to the beads, the mixture was agitated at 1,000 rpm for 2 hours and then washed three times with 250 µL of Exosome Immobilizing/Washing Buffer. Finally, the EVs captured on the beads were eluted with 100 mM Tris-HCl (pH 8.0) and 20 mM NaCl containing 4% SDS.

### Protein digestion

Protein lysate and purified EVs were treated with 20 mM tris(2-carboxyethyl)phosphine at 80°C for 10 min and were then alkylated with 35 mM iodoacetamide at room temperature for 30 min under light-shielded conditions. The treated samples were subjected to cleanup and digestion using the single-pot solid-phase-enhanced sample preparation (SP3) with TANBead Maelstrom 8 (TANBead, Taipei, Taiwan). Briefly, two types of Sera-Mag SpeedBead carboxylate-modified magnetic particles (hydrophilic particles, CAT# 45152105050250; hydrophobic particles, CAT# 65152105050250; Cytiva, Marlborough, MA, USA) were used. These beads were combined at a 1:1 (v/v) ratio, washed twice with distilled water, and reconstituted in distilled water at a concentration of 8 µg solids/µL. Then, 20 µL of the reconstituted beads was added to the alkylated protein sample, and 99.5% ethanol was added to achieve a final concentration of 75% (v/v). The mixture was mixed for 5 min. The supernatant was discarded, and the pellet was washed twice with 80% ethyl alcohol. The beads were resuspended in 100 µL of 50 mM Tris-HCl (pH 8.0) or in 50 mM Tris-HCl (pH 8.0) containing 0.02% LMNG. Next, 500 ng of trypsin/Lys-C Mix (CAT# V5072, Promega, Madison, WI, USA) was added, and the mixture was gently incubated at 37°C overnight to digest the proteins. The digested sample was acidified with 20 µL of 5% trifluoroacetic acid (TFA) and subjected to sonication at a high level at room temperature for 5 min using Bioruptor II (CosmoBio, Tokyo, Japan). The sample was desalted using either an SDB-STAGE tip (CAT# 7820-11200, GL Sciences Inc., Tokyo, Japan) or an Evotip Pure (CAT# EV2015, EVOSEP, Odense, Denmark). The SDB-STAGE tip was washed with 25 µl of 80% ACN in 0.1% TFA and equilibrated with 50 µl of 3% ACN in 0.1% TFA. The sample was loaded onto the tip, washed with 80 µl of 3% ACN in 0.1% TFA, and eluted with either 50 µL of 50% ACN in 0.1% TFA or 36% ACN in 0.1% TFA.

The eluate was dried in a centrifugal evaporator (miVac Duo concentrator). The dried sample was redissolved in 8 µL of 2% ACN containing 0.1% TFA or 0.01% DMNG and transferred to a normal vial. 4 µL of the sample was injected into the LC-MS/MS. The Evotip Pure was used according to the manufacturer's protocol. Briefly, the tip was washed with 20 µl of ACN in 0.1% formic acid (FA), followed by equilibration with 20 µl of ACN. The sample was loaded onto the tip and washed with 20 µl of H₂O in 0.1% FA. The tip was then filled with 0.1% in H₂O until LC-MS/MS measurements.

### Co-IP and on-beads digestion

IP lysis buffer [Pierce IP Lysis Buffer (CAT# 87788, Thermo Fisher Scientific, Waltham, MA, USA) containing a protease inhibitor (CAT# 5892791001, cOmplete ULTRA Tablets, Sigma-Aldrich, MO, USA) and a phosphatase inhibitor (CAT# 4906837001, PhosSTOP Tablets, Sigma-Aldrich)] was added to HeLa cells and mixed at 4°C for 30 min. The cell lysate was then centrifuged at 18,000g at 4 °C for 30 min and the supernatant was collected. The protein concentration of the protein extract was determined using a BCA protein assay kit (CAT# 23225, Thermo Fisher Scientific) and adjusted to 2 µg/µL with the IP lysis buffer. Co-immunoprecipitation (Co-IP) from the HeLa cell lysate was performed using the automated TANBead Maelstrom 8 (TANBead). For Co-IP, Sera-Mag SpeedBeads Protein A/G Magnetic Particles (CAT# 171521040150, Cytiva, Uppsala, Sweden) were used as the beads, and anti-RELA (NF-κB p65) antibody (CAT# ab16502, Abcam, Cambridge, MA, USA) was used as the antibody. To prepare the beads for immunoprecipitation, 1.5 µL of bead slurry was washed once with 500 µL of Tris-buffered saline containing 0.05% Tween20 (TBST). The beads were then mixed in 200 µL of TBST containing 4 µg of anti-RELA antibody at room temperature for 30 min, washed twice with 500 µL of TBST to remove unbound antibody, thereby immobilizing the antibody on the beads. Subsequently, 200 µL of HeLa cell lysate (2 µg/µL) was added to the beads, and the mixture was incubated at room temperature for 60 min with end-over mixing. The beads were then washed three times with 500 µL of the IP lysis buffer and twice with 500 µL of 50 mM Tris-HCl (pH 8.0). Thereafter, 100 µL of 50 mM Tris-HCl (pH 8.0) containing 0.02% LMNG or 50 mM Tris-HCl (pH 8.0) was added to the beads. Then, 500 ng of trypsin/Lys-C Mix (CAT# V5072, Promega) was added, and the mixture was gently mixed overnight at 37°C to digest the proteins. The beads were then aggregated from the digested sample using a magnetic stand (EpiMag HT (96-Well) Magnetic Separator, EpiGentek, Brooklyn, NY, USA), and the supernatant was collected. The collected sample was treated with 20 mM tris (2-carboxyethyl)phosphine at 80°C for 10 min and alkylated using 35 mM iodoacetamide at room temperature for 30 min under light-shielded conditions. The alkylated sample was then acidified with 10 µL of 10% TFA (total volume: 120 µL), desalted using an SDB-STAGE tip (eluent: 36% acetonitrile in 0.1% TFA), and dried in a centrifugal evaporator (miVac Duo concentrator). The dried sample was redissolved in 10 µL of 2% acetonitrile containing either 0.1% TFA or 0.01% DMNG and transferred to a normal vial (Thermo Fisher Scientific). One microliter of the sample was injected into an LC-MS/MS.

### Typical DDA-MS by LC-MS/MS

The redissolved peptides were directly injected onto a 75 µm × 12 cm nanoLC nano-capillary column (Nikkyo Technos Co., Ltd., Tokyo, Japan) at 50 °C and then separated with a 30 min gradient (A = 0.1% FA in water, B = 0.1% FA in 80% ACN) consisting of 0 min 5% B, 30 min 45% B at a flow rate of 300 nl/min using an UltiMate 3000 RSLCnano LC system (Thermo Fisher Scientific, Waltham, MA, USA). The peptides eluted from the column were analyzed on an Orbitrap Exploris 480 (Thermo Fisher Scientific) with DDA. MS1 spectra were acquired over the range of 380-1,240 m/z with a resolution of 60,000, an auto gain control (AGC) target of 3 × 10⁶, and a maximum injection time of 100 msec. The 50 most intense ions with charge states of 2+ to 5+ that exceeded 8.0 × 10³ were fragmented by collision-induced dissociation with a normalized collision energy of 28%. MS2 spectra were acquired in the range above 200 m/z with a resolution of 15,000, an AGC target of 1 × 10⁵ and a maximum injection time set to "Auto." The dynamic exclusion time was set to 30 seconds.

### Typical DIA-MS by LC-MS/MS

The redissolved peptides were directly injected onto a 75 µm × 12 cm nanoLC column (Nikkyo Technos Co., Ltd., Tokyo, Japan) at 50 °C and then separated with a 60 min gradient (A = 0.1% FA in water, B = 0.1% FA in 80% ACN) consisting of 0 min 8% B, 50 min 35% B, 57 min 70% B, 60 min 70% B at a flow rate of 200 nl/min using an UltiMate 3000 RSLCnano LC system. The peptides eluted from the column were analyzed on an Orbitrap Exploris 480 with DIA.

MS1 spectra were acquired over the range of 495-905 m/z with a resolution of 15,000, an automatic gain control target of 3 × 10⁶ and maximum injection time of 23 msec. MS2 spectra were acquired in the range above 200 m/z with a resolution of 30,000, an automatic gain control target of 3 × 10⁶, maximum injection time of "auto", and normalized collision energy of 26%. The isolation width for MS2 was set to 8 m/z, and for the 500-900 m/z window pattern, an optimized window arrangement was used in Xcalibur 4.4 (Thermo Fisher Scientific).

### EVOSEP ONE LC-MS/MS

The Evotip Pure sample was analyzed using an Orbitrap Exploris 480 mass spectrometer (Thermo Fisher Scientific, Waltham, MA, USA) equipped with an EVOSEP ONE system (EVOSEP) and an InSpIon system (AMR, Tokyo, Japan) with an electrospray ionization XYZ-stage and a column oven. EVOSEP ONE was acquired using Whisper 80 SPD method (gradient running time for 15 min at a flowrate of 100 nl/min). The digested peptides were separated using an Aurora Rapid 75 C18 capillary column (5 cm × 75 µm i.d., particle size 1.7 µm; IonOpticks, VIC, Australia) at 60 °C. The mobile phases were composed of 0.1% FA in H₂O (mobile phase A) and 0.1% FA in ACN (mobile phase B). The peptides eluted from the column were analyzed on an Orbitrap Exploris 480 with DIA. MS1 spectra were acquired in the range of 645-775 m/z with a resolution of 7,500, with an automatic gain control target of 1 × 10⁶ and maximum injection time of 10 msec. MS2 spectra were acquired in the range of 200-1,800 m/z with a resolution of 30,000, with an automatic gain control target of 3 × 10⁶, a maximum injection time set to "auto", and a normalized collision energy of 28%. The MS2 isolation window width was set to 8 m/z. For the 650-770 m/z window pattern, an optimized window arrangement was used in Xcalibur 4.4 (Thermo Fisher Scientific).

### Protein identification and quantitative analysis from MS data

The DDA-MS files were searched against the human protein sequence database (Proteome ID UP000005640, reviewed, canonical; 20,591 entries) using Proteome Discoverer 3.0 with Sequest HT and Percolator (Thermo Fisher Scientific). The search parameters were set as follows:

| | |
|---|---|
| Enzyme | Trypsin |
| Maximum missed cleavages | 2 |
| Precursor mass tolerance | 10 ppm |
| Fragment mass tolerance | 0.02 Da |
| Static modification | Cysteine carbamidomethylation |
| Dynamic modification | Methionine Oxidation |

The DIA-MS files were also searched against an in silico human spectral library using DIA-NN (version 1.8.1,
https://github.com/vdemichev/DiaNN) 14. First, a spectral library was generated from the human protein sequence database using DIA-NN. The parameters used for generating spectral library were as follows:

| | |
|---|---|
| Digestion enzyme | Trypsin |
| Missed cleavages | 1 |
| Peptide length range | 7-45 |
| Precursor charge range | 2-4 |
| Precursor m/z range | 395-1005 |
| fragment ion m/z range | 200-1800 |
| FASTA digest for library-free search/library generation | Enabled |
| Deep learning-based spectra, RTs, and IMs prediction | Enabled |
| n-term M excision | Enabled |
| C carbamidomethylation | Enabled |

The search parameters for DIA-NN are as follows:

| | |
|---|---|
| Mass accuracy | 10 ppm (15 ppm with Evosep One); |
| MS1 accuracy | 10 ppm (15 ppm with Evosep One) |
| Protein inference | Genes |
| Neural network classifies | Single-pass mode |
| Quantification strategy | Robust LC (high precision) |
| Cross-run normalization | Off |
| Unrelated runs | Enabled |
| Use isotopologues | Enabled |
| Heuristic protein inference | Enabled |
| No shared spectra enabled | Enabled |

MBR was turned off when searching for protein and precursor identifications and turned on for quantitative analysis. The threshold for protein identification was set at 1% or less for both precursor and protein FDRs.

### Phosphoproteomics

100 µg of peptide digests were dissolved in 80% ACN-0.1 TFA. The dissolved peptides were added to Fe beads washed with 80% CAN-0.1% TFA and mixed for 60 min at room temperature. The beads were then washed twice with 1 mL of 80% CAN-0.1% TFA and once with 1 mL of 0.1% TFA. Phosphopeptides were eluted by adding 200 µL of 3% polyphosphoric acid or 3% polyphosphoric acid containing 0.02% LMNG, followed by mixing for 10 min at room temperature. The eluted phosphopeptides were desalted with an SDB-STAGE tip (eluent: 36% ACN in 0.1% TFA). The dried samples were redissolved in 10 µL of 0.01% DMNG and transferred to normal vials. The dried peptides were directly injected onto a 75 µm × 30 cm nanoLC column (CAT# HEB07503001718IWF, CoAnn Technologies, Richland, WA, USA) at 60 °C. Peptides were separated using an UltiMate 3000 RSLCnano LC system (Thermo Fisher Scientific, Waltham, MA, USA) at a flow rate of 150 nL/min with a 100-min gradient (A: 0.1% FA in water; B: 0.1% FA in 80% ACN) consisting of 0 min at 5% B, 86 min at 35% B, 93 min at 70% B, and 100 min at 70% B.
Peptides eluted from the column were analyzed using an Orbitrap Exploris 480 (Thermo Fisher Scientific) in DDA mode. MS1 spectra were acquired over the range of 400-1,500 m/z with a resolution of 60,000 with an automatic gain control (AGC) target of 3 × 10⁶ and maximum injection time set to "Auto". The top 50 most intense ions with charge states of 2+ to 4+ that exceeded 2.0 × 10⁴ were fragmented by collision induced dissociation using stepped normalized collision energies of 22%, 26%, and 30%. MS2 spectra were collected over the range of 200-1,800 m/z with a resolution of 30,000, with an AGC target of 5 × 10⁵ and maximum injection time set to "Auto". The dynamic exclusion time was set to 30 seconds. MS files were searched against the human protein sequence database (proteome ID UP000005640, reviewed, canonical, 20,591 entries) using Proteome Discoverer 3.0 with Sequest HT and Percolator (Thermo Fisher Scientific). The parameters were set as follows:

| | |
|---|---|
| Enzyme | Trypsin |
| Maximum missed cleavage sites | 2 |
| Precursor mass tolerance | 10 ppm |
| Fragment mass tolerance | 0.02 Da |
| Static modification | Cysteine carbamidomethylation |
| Dynamic modification phosphorylation. | Serine, threonine and tyrosine |

### LC-MS/MS analysis of biotinylated BSA

A mixture of 10 ng of biotinylated BSA digest and 5 µg of K562 cell digest (Promega) was added to streptavidin beads (10 µL slurry was used; Cat# 211521040150, Cytiva) that had been washed once with TBST, and mixed for 60 min at room temperature. The beads were then washed three times with 1 mL of 50 mM Tris-HCl (pH 8.0) containing 0.5% SDS and 500 mM NaCl, followed by a single wash with 1 mL of TBS. The beads were added with 50 µL of 8M guanidine-HCl (pH 1.5) containing 0.5 mM biotin or 8M guanidine-HCl (pH 1.5) containing 0.02% LMNG and 0.5 mM biotin, and mixed for 2 hours at room temperature to elute the biotinylated peptides. The eluted peptides were desalted using SDB-STAGE tips (elution: 36% ACN in 0.1% TFA). The dried sample was redissolved in 10 µL of 0.01% DMNG, and transferred to a normal vial. The redissolved peptides were directly injected onto a 75 µm × 12 cm nanoLC nano-capillary column (Nikkyo Technos, Tokyo, Japan) at 50 °C and separated at a flow rate of 300 nL/min using an UltiMate 3000 RSLCnano LC system (Thermo Fisher Scientific, Waltham, MA, USA) with a 30 min gradient (A = 0.1% FA in water; B = 0.1% FA in 80% ACN) consisting of 0 min at 8% B, 26 min at 45% B, 29 min at 85% B, and 30 min at 85% B. The peptides eluted from the column were analyzed on a Q Exactive HF-X mass spectrometer (Thermo Fisher Scientific) equipped with an InSpIon system. MS1 spectra were acquired over the range of 450-1,500 m/z with a resolution of 120,000 with an automatic gain control (AGC) target of 3 × 10⁶ and a maximum injection time of 100 ms. The 20 most intense precursor ions with charge states from 2+ to 7+ that exceeded 8.0 × 10³in intensity were fragmented by collision-induced dissociation using stepped normalized collision energies of 22%, 26%, and 30%. MS2 spectra were acquired over a range above 200 m/z with a resolution of 60,000 with an AGC target of 2 × 10⁵ and a maximum injection time of 120 ms. The dynamic exclusion duration was set to 30 seconds. The MS files were searched against the human UniProt protein database (Proteome ID: UP000005640; 20,591 entries; downloaded on May 5, 2023) and BSA (UniProt Accession: P02769) using PEAKS Studio 11 (Bioinformatics Solution Inc., Waterloo, Canada). The MS files were searched against the human UniProt protein database (Proteome ID: UP000005640; 20,591 entries; downloaded on May 5, 2023) and BSA (UniProt Accession: P02769) using PEAKS Studio 11 (Bioinformatics Solution Inc., Waterloo, Canada). The parameters were set as follows:

| | |
|---|---|
| Enzyme | Trypsin |
| Maximum missed cleavage sites | 4 |
| Precursor mass tolerance | 10 ppm |
| Fragment mass tolerance | 0.02 Da |
| Static modification | Cysteine carbamidomethylation |
| Dynamic modification | Lysine biotinylation |

The threshold for protein identification was set at 1% or less for both peptide and protein FDRs.

### Data Analysis

Pearson correlation coefficient heat map analysis with principal component analysis (PCA) and hierarchical clustering was performed using Perseus v1.6.15.0.

### (Results and Discussion)

The surfactants listed in Table 6 were separated on a reversed-phase (C18) column and their elution times were compared. The results are shown in Figure 6.

**[Table 6]**

| Type of Sugar-Based Surfactants | Sugar-Based Surfactants | Abbreviation | Cas no |
|---|---|---|---|
| Maltose type | n-Dodecyl- *β* -D-maltoside | DDM | 69227-93-6 |
| Maltose type | n-Undecyl-*β* -D-maltoside | UDM | 253678-67-0 |
| Maltose type | n-Decyl- *β* -D-maltoside | DeDM | 82494-09-5 |
| Maltose type | n-Octyll- *β* -D-maltoside | ODM | 82494-08-4 |
| Maltose type | n-Nonyl- *β* -D-thiomaltoside | NDT | 148565-55-3 |
| Glucose type | n-Octyl- *β* -D-glucoside | ODG | 29836-26-8 |
| Glucose type | n-Heptyl- *β* -D-thioglucoside | HDT | 85618-20-8 |
| Glucose type | n-Octyl- *β* -D-thioglucoside | ODT | 85618-21-9 |
| Mannose type | 3-Oxatridecyl- *α* -D-mannoside | 3ODM | 914802-92-9 |
| Neopentyl Glycol | Decyl Maltose Neopentyl Glycol | DMNG | 1257852-99-5 |
| Neopentyl Glycol | Lauryl Maltose Neopentyl Glycol | LMNG | 1257852-96-2 |
| Neopentyl Glycol | Octyl Glucose Neopentyl Glycol | OMNG | 1257853-32-9 |
| Trehalose ester type | Trehalose C12 | TC12 | 64622-91-9 |

As a result, it was confirmed that LMNG had an obviously delayed elution time compared to other surfactants, indicating that LMNG has a strong affinity for reversed-phase columns. Since LMNG has an extremely strong affinity for reversed-phase columns, it was suggested that, when LMNG is added for the purpose of peptide affinity for LC-MS analysis or suppression of adsorption to tubes, LMNG is not eluted from the reversed-phase solid-phase column even after peptides are eluted, and therefore LMNG can be readily removed, when desalting the sample with the reversed-phase solid-phase column.

Subsequently, elution conditions from reversed-phase solid-phase extraction columns were examined for three surfactants, LMNG, DMNG, and DDM, which showed the latest elution times. The reversed-phase solid-phase extraction column with SDB carriers used in the evaluation is generally used for desalting digested peptides, and 50% to 80% ACN solution is typically used as an eluent to ensure peptide elusion from the column. Therefore, LMNG, DMNG and DDM were trapped on the SDB solid-phase extraction column and eluted sequentially with increasing organic solvent concentration using 30% ACN-0.1% TFA, 40% ACN-0.1% TFA, 50% ACN-0.1% TFA, and 60% ACN-0.1% TFA, and the eluates were analyzed by LC-MS to determine the elution times. As a result, DDM and DMNG were eluted with 30% ACN-0.1% TFA (Figure 7). Therefore, in order to elute digested peptides while retaining these surfactants adsorbed on the reversed-phase solid-phase column, it is necessary to use an ACN concentration lower than 30%. However, when the ACN concentration is lower than 30%, hydrophobic peptides that have high affinity for the reversed-phase carrier may not be sufficiently eluted and may be lost, posing a significant risk. On the other hand, LMNG was not eluted at all with 30% ACN-0.1% TFA and was only slightly eluted with 40% ACN-0.1% TFA (Figure 7). Further examination of the elution condition of LMNG revealed that it was not eluted with ACN-0.1% TFA at concentrations of 38% or less (Figure 8). Since the elution behavior on a solid-phase extraction column may slightly vary depending on temperature and elution speed, 36% ACN-0.1% TFA may be adopted as a condition under which LMNG is not eluted, with a safety margin. Next, whether 36% ACN-0.1% TFA is sufficient as an elution condition for digested peptides was evaluated. As a result, the number of peptides identified under the elution conditions with 50% ACN-0.1% TFA and 36% ACN-0.1% TFA showed almost no difference (Figure 9), indicating that peptide components were nearly completely eluted from the reversed-phase solid-phase column with 36% ACN-0.1% TFA. From the above, the present inventors successfully established a method for removing LMNG without affecting the elution of peptides from reversed-phase solid-phase extraction columns. Since LMNG can be removed easily, whether peptide loss could be improved by adding LMNG to actual digested peptide samples was examined. Specifically, 100 ng of HEK293 cell lysate was used as the starting material, and it was confirmed that the number of peptides identified by LC-MS dramatically increased when LMNG was added during trypsin digestion, compared to when LMNG was not added (Figure 10). An increase in the number of identified peptides was observed at LMNG concentrations of 0.01% to 0.04%, with the highest number of peptides identified at a concentration of 0.02%.

Furthermore, the present inventors attempted to minimize sample loss by adding LMNG to the digested peptide solution, desalting the solution with a reversed-phase solid-phase extraction column, drying the eluted peptides, and dissolving the dried peptides in an aqueous solution of low-concentration DMNG (Figures 11 and 12). In FIG. 11, 100 ng of HEK293 cell lysate was used as a starting material. The addition of LMNG to the digestion solution significantly improved the number of precursors (peptides) and proteins identified by LC-MS, and further improvement in these identification numbers was observed when the dried peptides were dissolved in DMNG. In addition, in the sample without LMNG, individual protein quantification values varied greatly, resulting in a low Pearson correlation coefficient r. For trace samples such as 100 ng of HEK293 cell lysate, it was suggested that the addition of LMNG not only improves the number of identified proteins and precursors (peptides), but also improves the reproducibility of the pretreatment. Furthermore, the effect of adding LMNG during on-bead digestion in co-immunoprecipitation mass spectrometry (coIP-MS), as well as the effect of dissolving dried peptides with DMNG, were investigated. In Figure 12, coIP-MS was performed using an anti-RELA antibody. As in the experiment in Figure 11, the addition of LMNG during digestion significantly improved the number of identified precursors (peptides) and proteins, and further improvement in these identification numbers was achieved by dissolving the dried peptides with DMNG. The recovery of RELA, which is the antigen recognized by the antibody, and NFKB1, IKBKB, NFKBIA, NFKBIB and NFKBIE, , which are known to interact with RELA, was significantly improved by adding LMNG during digestion, and was further improved by adding DMNG during dissolution of the dried peptides. From these results, it was confirmed that the addition of LMNG during digestion and the addition of DMNG during dissolution of dried peptides also have beneficial effects in coIP-MS. Furthermore, the number of identified phosphopeptides was improved by adding LMNG to the eluent during enrichment of phosphopeptides by immobilized metal ion affinity chromatography (Figures 13 and 14). In addition, by adding LMNG to the eluent during concentration of biotinylated peptides using avidin beads, the recovery of biotinylated peptides was increased and the number of identified biotinylated peptides was improved (Figures 15 and 16). Since LMNG can be easily removed by a reversed-phase solid-phase extraction column, it is applicable not only to peptide loss during digestion, but also to affinity purification of peptides, and wide applicability is expected.

Finally, we investigated the applicability of LMNG to analysis using EVOSEP ONE, a LC specialized for carryover-free multi-sample analysis developed for clinical proteome analysis. EVOSEP ONE is an LC device widely used around the world for proteome analysis of several hundred to several thousand samples. In EVOSEP ONE, samples are trapped on a reversed-phase solid-phase extraction column (Evotip Pure (C18)) and then set it in EVOSEP ONE to allow LC analysis. In EVOSEP ONE, an optimized method for peptide analysis has already been prepared by the manufacturer, and the detailed gradient settings cannot be modified. First, the present inventors examined whether LMNG can be eluted using EVOSEP ONE. As controls, DDM and DMNG were trapped on Evotip Pure, separated using EVOSEP ONE, and measured with MS (Figure 17). Peaks were detected at the final part of the elution time for both DDM and DMNG, indicating that they were eluted from Evotip Pure, whereas elution of LMNG was not observed. When 100 ng of HEK293 cell lysate was digested with or without LMNG and analyzed by LC-MS/MS using EVOSEP ONE, a greater number of precursors (peptides) and proteins were identified with LMNG (Figure 18). In addition, even when exosomes were purified from serum samples, which are often subjected to multi-sample analysis, and analyzed by LC-MS/MS using EVOSEP ONE, the addition of LMNG during digestion improved the identification of precursors (peptides) and proteins and increased the recovery of representative exosome markers such as CD9, CD63, and CD81 (Figure 19). LMNG increased the recovery of peptides also in EVOSEP ONE and can be easily removed by Evotip pure, confirming its high applicability to high-throughput proteome analysis using

### EVOSEP ONE.

In the examples herein, the concentration of acetonitrile, trifluoroacetic acid, and formic acid in a solution expressed as percentage means volume/volume percent, and the concentration of a surfactant in a solution expressed as percentage means weight/weight percent.

### INDUSTRIAL APPLICABILITY

The present invention provides a surfactant that can suppress the loss of peptides and be easily removed in the pretreatment of samples for proteome analysis. By treating a sample for proteome analysis using the method of the present invention, the loss of peptides from trace samples is suppressed, the number of peptides and proteins identified by mass analysis is increased, and the reproducibility of analysis is improved. In addition, according to the present invention, the recovery of peptide fragments adsorbed onto the tube wall by drying during the pretreatment process for proteome analysis is improved. In particular, since the recovery of long peptides with a length of 11 amino acids or more is greatly improved, it is possible to prepare a sample for mass spectrometry that is rich in long peptides, thereby increasing the number of peptides and proteins identified by mass spectrometry and contributing to improved accuracy of proteome analysis. Since the effect of improved recovery of peptide fragments can be observed at a very low concentration of sugar-based nonionic surfactants, contamination of the mass spectrometer can also be minimized. Furthermore, according to the present invention, a stable aqueous peptide solution in which adsorption of peptides to the vessel is suppressed.

This application is based on a patent application Nos. 2022-199022 (filing date: December 13, 2022) and 2023-131671 (filing date: August 10, 2023) filed in Japan, the contents of which are incorporated in full herein.

## Claims

1. A method for preparing a peptide-containing sample comprising the following steps:
1) providing an aqueous peptide solution containing a peptide and 2,2-dodecylpropane-1,3-bis-β-D-maltopyranoside (hereinafter referred to as "LMNG");
2) applying the obtained aqueous peptide solution to a reversed-phase column to allow the peptide and LMNG to be adsorbed onto the reversed-phase column; and
3) eluting the adsorbed peptide from the reversed-phase column while retaining LMNG adsorbed onto the reversed-phase column to remove LMNG from the peptide.

2. The preparation method according to claim 1, wherein the reversed-phase column is a C18 column or a styrene-divinylbenzene copolymer column.

3. The preparation method according to claim 1, wherein the peptide is eluted from the reversed-phase column with an eluent having a polarity equivalent to or greater than a mixed solution of acetonitrile / 0.1% (v/v) trifluoroacetic acid aqueous solution = 50/50 (v/v).

4. The preparation method according to claim 3, wherein the polarity of the eluent is equivalent to or less than a mixed solution of acetonitrile / 0.1% (v/v) trifluoroacetic acid aqueous solution = 30/70 (v/v).

5. The preparation method according to claim 1, wherein the LMNG concentration in the aqueous peptide solution is lower than the critical micelle concentration.

6. The preparation method according to claim 1, wherein the aqueous peptide solution is provided as contained in a vessel having a plastic surface.

7. The preparation method according to claim 1, further comprising subjecting a protein dissolved in an aqueous solvent containing LMNG to limited digestion with a protease to obtain an aqueous peptide solution containing a peptide and LMNG.

8. The preparation method according to claim 1, further comprising applying the aqueous peptide solution provided in step 1) to an affinity chromatography to obtain an aqueous peptide solution containing an affinity-enriched peptide and LMNG.

9. The preparation method according to claim 8, wherein the affinity chromatography is immobilized metal affinity chromatography or avidin or streptavidin affinity chromatography.

10. The preparation method according to claim 1, wherein the peptide-containing sample is for LC-MS analysis.

11. A method for producing an aqueous peptide solution, comprising dissolving a dried peptide in an aqueous solvent containing at least one sugar-based nonionic surfactant.

12. The production method according to claim 11, wherein the sugar-based nonionic surfactant is a compound represented by formula (I);
[Chemistry 1]
G₁ - L₁ - R₁ (I)
(wherein G₁ is a monosaccharide or disaccharide residue, Li is O, S, -O-(CH₂)ₙ-O-, or -O-CO-, R₁ is a linear alkyl group having 6 to 12 carbon atoms, and n is an integer of 1 to 3), or by formula (II); 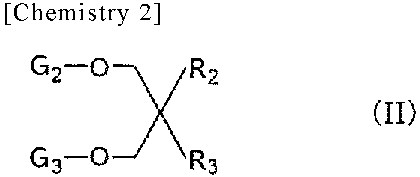 (wherein G₂ and G₃ are the same or different and represent a monosaccharide or disaccharide residue, and R₂ and R₃ are the same or different and represent a linear alkyl group having 6 to 12 carbon atoms).

13. The production method according to claim 12, wherein the sugar-based nonionic surfactant is a compound represented by formula (I), and G₁ is glucose residue, mannose residue, maltose residue or trehalose residue.

14. The production method according to claim 13, wherein the sugar-based nonionic surfactant is n-dodecyl-β-D-maltoside, n-undecyl-β-D-maltoside, n-decyl-β-D-maltoside, n-nonyl-β-D-thiomaltoside, n-octyl-β-D-thioglucoside, 3-oxatridecyl-α-D-mannoside, or α-D-glucopyranosyl-α-D-glucopyranoside monododecanoate.

15. The production method according to claim 12, wherein the sugar-based nonionic surfactant is a compound represented by formula (II), and G₂ and G₃ are the same and represent glucose residue or maltose residue.

16. The production method according to claim 15, wherein the sugar-based nonionic surfactant is 2,2-dioctylpropane-1,3-bis-β-D-maltopyranoside, 2,2-didecylpropane-1,3-bis-β-D-maltopyranoside, or 2,2-dihexylpropane-1,3-bis-β-D-glucopyranoside.

17. The production method according to claim 11, wherein the aqueous solvent contains a combination of at least two sugar-based nonionic surfactants.

18. The production method according to claim 17, wherein the combination of sugar-based nonionic surfactants is selected from the group consisting of:
n-dodecyl-β-D-maltoside and α-D-glucopyranosyl-α-D-glucopyranoside monododecanoate;
2,2-dihexylpropane-1,3-bis-β-D-glucopyranoside and α-D-glucopyranosyl-α-D-glucopyranoside monododecanoate;
2,2-dioctylpropane-1,3-bis-β-D-maltopyranoside and 2,2-dihexylpropane-1,3-bis-β-D-glucopyranoside; and
3-oxatridecyl-α-D-mannoside and 2,2-dioctylpropane-1,3-bis-β-D-maltopyranoside.

19. The production method according to claim 11, wherein the dried peptide is contained in a vessel having a plastic surface.

20. An agent for accelerating the dissolution of a dried peptide, comprising at least one sugar-based nonionic surfactant.

21. The agent for accelerating the dissolution of a dried peptide according to claim 20, comprising a combination of at least two sugar-based nonionic surfactants.

22. The agent for accelerating the dissolution of a dried peptide according to claim 21, wherein the combination of sugar-based nonionic surfactants is selected from the following group:
n-dodecyl-β-D-maltoside and α-D-glucopyranosyl-α-D-glucopyranoside monodododecanoate;
2,2-dihexylpropane-1,3-bis-β-D-glucopyranoside and α-D-glucopyranosyl-α-D-glucopyranoside monodododecanoate;
2,2-dioctylpropane-1,3-bis-β-D-maltopyranoside and 2,2-dihexylpropane-1,3-bis-β-D-glucopyranoside; and
3-oxatridecyl-α-D-mannoside and 2,2-dioctylpropane-1,3-bis-β-D-maltopyranoside.

23. An aqueous peptide solution, comprising a peptide and a combination of at least two sugar-based nonionic surfactants.

24. The aqueous peptide solution according to claim 23, wherein the combination of sugar-based nonionic surfactants is any one selected from the following group:
n-dodecyl-β-D-maltoside and α-D-glucopyranosyl-α-D-glucopyranoside monodododecanoate;
2,2-dihexylpropane-1,3-bis-β-D-glucopyranoside and α-D-glucopyranosyl-α-D-glucopyranoside monodododecanoate;
2,2-dioctylpropane-1,3-bis-β-D-maltopyranoside and 2,2-dihexylpropane-1,3-bis-β-D-glucopyranoside; and
3-oxatridecyl-α-D-mannoside and 2,2-dioctylpropane-1,3-bis-β-D-maltopyranoside.

25. The preparation method according to claim 1, further comprising the following steps:
4) drying the eluted peptide to obtain a dried peptide, and
5) dissolving the obtained dried peptide in an aqueous solvent containing at least one sugar-based nonionic surfactant to obtain an aqueous peptide solution.
